Europäisches Patentamt

European Patent Office

Office européen des brevets

(19)

(11) **EP 1 542 149 A1**

(12) **EUROPEAN PATENT APPLICATION**
published in accordance with Art. 158(3) EPC

(43) Date of publication:
15.06.2005 Bulletin 2005/24

(21) Application number: 03771398.9

(22) Date of filing: 29.07.2003

(51) Int Cl.⁷: $G06F\ 19/00$

(86) International application number:
PCT/JP2003/009579

(87) International publication number:
WO 2004/012128 (05.02.2004 Gazette 2004/06)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 30.07.2002 JP 2002222192
13.12.2002 JP 2002361545

(71) Applicant: YAMANOUCHI PHARMACEUTICAL
CO. LTD.
Tokyo 103-8411 (JP)

(72) Inventors:
• UEDA, Hiroki,
YAMANOUCHI PHARMAC., CO., LTD.
Tsukuba-shi, Ibaraki 305-8585 (JP)
• HASHIMOTO, Seiichi,
YAMANOUCHI PHARMAC., CO., LTD.
Tsukuba-shi, Ibaraki 305-8585 (JP)

(74) Representative: Rupprecht, Kay, Dipl.-Ing. et al
Meissner, Bolte & Partner
Postfach 86 06 24
81633 München (DE)

(54) **APPARATUS FOR FORMING MOLECULAR TIMETABLE AND APPRATUS FOR ESTIMATING CIRCADIAN CLOCK**

(57) An apparatus 11 for estimating internal biological time of a biological individual on the basis of quantity data of a gene product as measured in a specimen sampled from the individual.

The apparatus includes circadian oscillatory gene selection means for selecting circadian oscillatory genes whose time-course change in gene product quantity data as measured in standard specimens approximates a cosine curve having a predetermined period; circadian expression curve selection means for selecting, from among a plurality of cosine curves having different phases and sharing a specific period, a circadian expression curve which is similar to the pattern of time-course change in the expression product quantity of each of the above-selected circadian oscillatory genes; and registration means for registering information which identifies the selected circadian expression curve.

Fig. 6

**Description**

Technical Field

The present invention relates to a molecular timetable generating apparatus, an internal biological time estimating apparatus, a molecular timetable generation method, an internal biological time estimating method, a molecular timetable generating program, an internal biological time estimating program, and an internal biological time estimating system. In particular, the invention relates to a molecular timetable generating apparatus, an internal biological time estimating apparatus, a molecular timetable generation method, an internal biological time estimating method, a molecular timetable generating program, an internal biological time estimating program, and an internal biological time estimating system; the apparatuses, methods, programs, and the system facilitating estimation of internal biological time, by use of a computer, from results of a test performed on a specimen obtained from single-time-point sampling.

Background Art

Many organisms have internal biological clocks, which control various biological rhythms such as sleep and wakefulness rhythm, blood-pressure rhythm, body-temperature rhythm, and some hormonal secretion rhythms.

The oscillation cycle of internal biological clocks is approximately 24 hours, but the cycle slightly differs from biological species to species. In the case of humans, the oscillation cycle is said to be about 25 hours. A rhythm of around 24 hours in a steady environment which involves no cyclic change is called "circadian rhythm." In daily life, sunlight serves as the strongest synchronization factor for synchronizing, day by day, the circadian rhythm with a 24-hour cycle.

An anomaly in circadian rhythm is a known cause of sleep-wake rhythm disorders (e.g., delayed sleep phase syndrome, advanced sleep phase syndrome, non-24-hour sleep-wake rhythm syndrome), seasonal depression, jet lag syndrome, sleep disturbance experienced by day/night shift workers, nighttime wandering of aged persons suffering dementia, and other pathological conditions such as delirium, as well as a cause of school truancy or refusal to go to work.

Moreover, the time of day a person takes a medicine (medicine intake time) is known to affect the efficacy of the medicine, strength of its side effects, and pharmacokinetics. The internal biological time of a night worker, a shift worker, or the like is sometimes different from that of ordinary persons. Therefore, when such a person takes a medicine at a timing suitable for ordinary persons, the medicine is not expected to provide a sufficient effect unless the timing matches his circadian time suitable for taking the medicine.

Accordingly, for the purpose of, for example, diagnosis of persons suffering circadian rhythm disorders, determination of medicine intake times on an individual basis, or prevention of nighttime wandering of aged persons suffering dementia, there has been demand for development of a system capable of determining an individual's internal biological time on an individual basis and offering information on the individual's internal biological time to the neurological departments of medical facilities, medical doctors who prescribe medicines, hospitals for the elderly, and health care facilities for the elderly. Thus, research on a method for estimating internal biological time is being developed.

Conceivable methods for estimating internal biological time include a method in which blood is drawn from an individual over time to thereby measure the blood melatonin level, and the individual's internal biological time is estimated on the basis of the peak melatonin level and the time at which the peak level is obtained; a method in which activity level and sleep of an individual is measured over time for determining the sleep-wake rhythm of the individual, whereby the individual's internal biological time is estimated; and a method in which the body temperature of an individual is measured so as to determine the body temperature rhythm of the individual, whereby the individual's internal biological time is estimated.

However, these methods have a drawback in that drawing of blood, body-temperature measurement, or the like must be performed as time elapses over 24 hours or more, which imposes a great burden on both patients and medical facilities. Therefore, these are not practical methods suitable for use in actual medical sites.Meanwhile, in some work related to time-course measurement of the quantity of gene products of drosophila heads by use of oligo-DNA arrays (McDonald, M. and Rosbash, M., Cell, 107, 567-578.(2001), Claridge-Chang, A. *et al*., Neuron, 32, 657-671(2001)) or to investigation of circadian oscillatory genes in mouse liver by use of the differential display method (Kornmann, B. *et al*., Nucleic Acids Res., 29(11), e51 (2001)), many circadian oscillatory genes have been identified in relevant tissues, and in addition, times at which respective circadian oscillatory genes exhibit the maximum expression levels have also been reported.

However, no method for determining internal biological time by use of data obtained from existing methods as described above is known, and there still remains demand for establishment of a method for measuring internal biological time.

An object of the present invention is to solve the above problem by provision of a molecular timetable generating apparatus, an internal biological time estimating apparatus, a molecular timetable generation method, an internal biological time estimating method, a molecular timetable generating program, an internal biological time estimating program, and an internal biological time estimating system; the apparatuses, methods, programs; and the system facilitating estimation of an individual's internal biological time through one drawing of a specimen; i.e., without need for a plurality of times of specimen collection.

Another object of the present invention is to provide a molecular timetable generating apparatus, an internal biological time estimating apparatus, a molecular timetable generation method, an internal biological time estimating method, a molecular timetable generating program, an internal biological time estimating program, and an internal biological time estimating system; the apparatuses, methods, programs, and the system facilitating estimation of an individual's internal biological time enabling provision of results of estimation of an individual's internal biological time to a client such as a medical facility, a health care facility for the elderly, and a sports club.

Disclosure of the Invention

As defined in claim 1 of the present invention, the molecular timetable generating apparatus of the present invention is an apparatus for generating a molecular timetable for estimating internal biological time of a biological individual on the basis of quantity data of a gene product (hereinafter referred to as gene product quantity data) as measured in standard specimens sampled from individuals, the apparatus comprising: data input means for inputting gene product quantity data of standard specimens each sampled from a predetermined portion of each of a plurality of individuals of a predetermined biological species; circadian oscillatory gene selection means for selecting, from among the genes which are expressed in the aforementioned standard specimens, circadian oscillatory genes whose time-course change in gene product quantity data approximates a cosine curve having a predetermined period; circadian expression curve selection means for selecting, from among a plurality of cosine curves having different phases and sharing a specific period, a circadian expression curve which is similar to the pattern of time-course change in the expression product quantity of each of the above-selected circadian oscillatory genes; and registration means for registering information which identifies the selected circadian expression curve.

Some of the above-mentioned objects are attained by a molecular timetable generating method as defined in claim 4, which enables, by use of information processing equipment, generation of a molecular timetable for estimating internal biological time of a biological individual on the basis of gene product quantity data as measured in standard specimens sampled from individuals, the method comprising: a data input procedure for inputting gene product quantity data of standard specimens each sampled from a predetermined portion of each of a plurality of individuals of a predetermined biological species; a circadian oscillatory gene selection procedure for selecting, from among the genes which are expressed in the aforementioned standard specimens, circadian oscillatory genes whose time-course change in gene product quantity data approximates a cosine curve having a predetermined period; a circadian expression curve selection procedure for selecting, from among a plurality of cosine curves having different phases and sharing a specific period, a circadian expression curve which is similar to the pattern of time-course change in the expression product quantity of each of the above-selected circadian oscillatory genes; and a registration procedure for registering information which identifies the selected circadian expression curve.

Some of the above-mentioned objects are attained by a molecular timetable generating program as defined in claim 6, which enables information processing equipment to perform: a data input procedure for inputting gene product quantity data of standard specimens each sampled from a predetermined portion of each of a plurality of individuals of a predetermined biological species; a circadian oscillatory gene selection procedure for selecting, from among the genes which are expressed in the aforementioned standard specimens, circadian oscillatory genes whose time-course change in gene product quantity data approximates a cosine curve having a predetermined period; a circadian expression curve selection procedure for selecting, from among a plurality of cosine curves having different phases and sharing a specific period, a circadian expression curve which is similar to the pattern of time-course change in the expression product quantity of each of the above-selected circadian oscillatory genes; and a registration procedure for registering information which identifies the selected circadian expression curve; said information processing equipment being provided for generation of a molecular timetable for estimating internal biological time of the biological individual on the basis of gene product quantity data as measured in standard specimens sampled from individuals.

Some of the above-mentioned objects are attained by an internal biological time estimation system as defined in claim 8 for enabling generation of a molecular timetable for estimating internal biological time of a biological individual on the basis of gene product quantity data as measured in standard specimens sampled from individuals and estimation of internal biological time of the biological individual on the basis of gene product quantity data as measured in a specimen sampled from the biological individual; said system comprising: a server computer installed in an information center for providing internal biological time information, and a terminal computer connected to the server computer so as to transmit and receive information therebetween, wherein the server computer includes: standard data input means for inputting gene product quantity data of standard specimens each sampled from a predetermined portion of each of a plurality of individuals of a predetermined biological species; circadian oscillatory gene selection means for selecting, from among the genes which are expressed in the aforementioned standard specimens, circadian oscillatory genes whose time-course change in gene product quantity data approximates a cosine curve having a predetermined period; circadian expression curve selection means for selecting, from among a plurality of cosine curves having different phases and sharing a specific period, a circadian expression curve which is similar to the pattern of time-course change in the expression product quantity of each of the above-selected circadian oscillatory genes; registration means for registering, as a standard molecular time of the circadian oscillatory gene, a point in time at which the circadian expression curve reaches the maximum, in the molecular timetable used for estimating the internal biological time, and also for registering, in the molecular timetable, an average value and standard deviation, both calculated for each circadian oscillatory gene, of the expression product quantity data, as a standard expression quantity and standard variation of the circadian oscillatory gene, respectively; measurement data input means for inputting the gene product quantity data of the circadian oscillatory genes contained in the specimen sampled from the said predetermined portion of the biological individual; internal biological time derivation means for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable; and internal biological time transmission means for transmitting the obtained internal biological time information to the terminal computer.

Thus, the present invention enables generation of timetables each of which specifies a circadian expression curve of a circadian oscillatory gene contained in a predetermined portion of a predetermined biological species, due to the provision of circadian oscillatory gene selection means for selecting, from among the genes which are expressed in the aforementioned standard specimens, circadian oscillatory genes whose time-course change in gene product quantity data approximates a cosine curve having a predetermined period; circadian expression curve selection means for selecting, from among a plurality of cosine curves having different phases and sharing a specific period, a circadian expression curve which is similar to the pattern of time-course change in the expression product quantity of each of the above-selected circadian oscillatory genes; and registration means for registering information which identifies the selected circadian expression curve. In other words, the invention enables provision, for an individual belonging to the biological species, of a timetable which serves as the basis for determining as to whether or not that individual has a circadian rhythm disorder, and when the individual does not have a circadian rhythm disorder, estimating the internal biological time of that individual.

Preferably, the registration means is configured so as to register, as a standard molecular time of the circadian oscillatory gene, a point in time at which the circadian expression curve reaches the maximum, in the molecular timetable used for estimating the internal biological time, and also to register, in the molecular timetable, an average value and standard deviation, both calculated for each circadian oscillatory gene, of the expression product quantity data, as a standard expression quantity and standard variation of the circadian oscillatory gene, respectively.

With this configuration, the present invention enables generation of a molecular timetable which specifies a circadian expression curve of a circadian oscillatory gene contained in a predetermined portion of a predetermined biological species. In other words, the invention enables provision, for an individual belonging to the biological species, of a molecular timetable which may be used for determining as to whether or not that individual has a circadian rhythm disorder, and when the individual does not have a circadian rhythm disorder, estimating the internal biological time of that individual. Moreover, registration of a circadian expression curve can be attained by use of the following data only; i.e., standard molecular time, standard expression level, and standard variation. Thus, circadian expression curves can be managed and controlled using a smaller number of data.

Some of the above-mentioned objects are attained by an internal biological time estimation apparatus as defined in claim 3 for estimating internal biological time of a biological individual on the basis of gene product quantity data as measured in specimens sampled from individuals, the apparatus comprising: molecular timetable storage means for storing molecular timetables each of which specifies a circadian expression curve showing time-course change in the expression product quantity of a circadian oscillatory gene contained in a predetermined portion of a predetermined biological species; data input means for inputting the gene product quantity data of the circadian oscillatory genes contained in a specimen sampled from said predetermined portion of the biological individual; and internal biological time derivation means for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable.

Some of the above-mentioned objects are attained by an internal biological time estimation method as defined in claim 5 for estimating, by use of information processing equipment, internal biological time of a biological individual on the basis of gene product quantity data as measured in specimens sampled from individuals, the method comprising: a molecular timetable storage procedure for storing molecular timetables each of which specifies a circadian expression curve showing time-course change in the expression product quantity of a circadian oscillatory gene contained in a predetermined portion of a predetermined biological species; a data input procedure for inputting the gene product quantity data of the circadian oscillatory genes contained in a specimen sampled from said predetermined portion of the biological individual; and an internal biological time derivation procedure for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable.

Some of the above-mentioned objects are attained by an internal biological time estimation program as defined in claim 7, which enables information processing equipment to perform: a molecular timetable storage procedure for storing molecular timetables each of which specifies a circadian expression curve showing time-course change in the expression product quantity of a circadian oscillatory gene contained in a predetermined portion of a predetermined biological species; a data input procedure for inputting the gene product quantity data of the circadian oscillatory genes contained in a specimen sampled from said predetermined portion of the biological individual; and an internal biological time derivation procedure for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable; said information processing equipment being provided for estimating internal biological time of the biological individual on the basis of gene product quantity data as measured in standard specimens sampled from individuals.

As described above, with an internal biological time derivation means for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable, a comparison of assay data on a specimen sampled at a single point in time with the above-mentioned circadian expression curves specified by molecular timetables generated for a predetermined portion of a predetermined biological species achieves, without need for sampling specimens a plurality of times, a simple judgment procedure for determining, on the basis of a specimen sampled at a single point in time, as to the presence or absence of a circadian rhythm disorder, and for estimating the internal biological time.

Brief Description of the Drawings

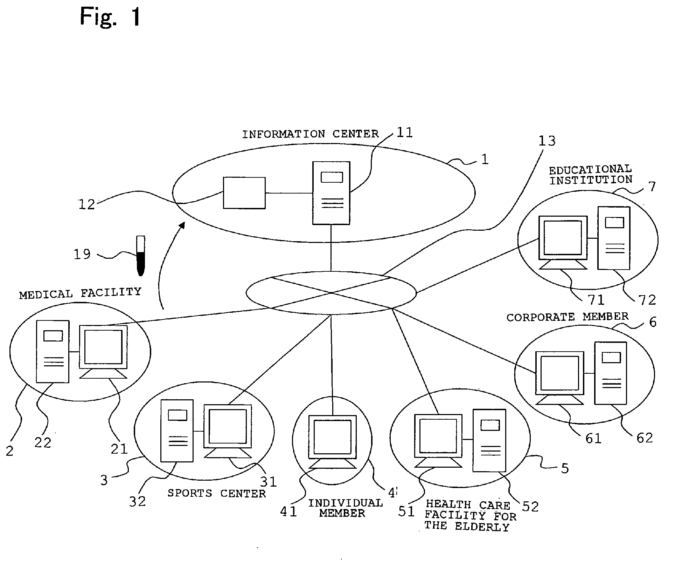

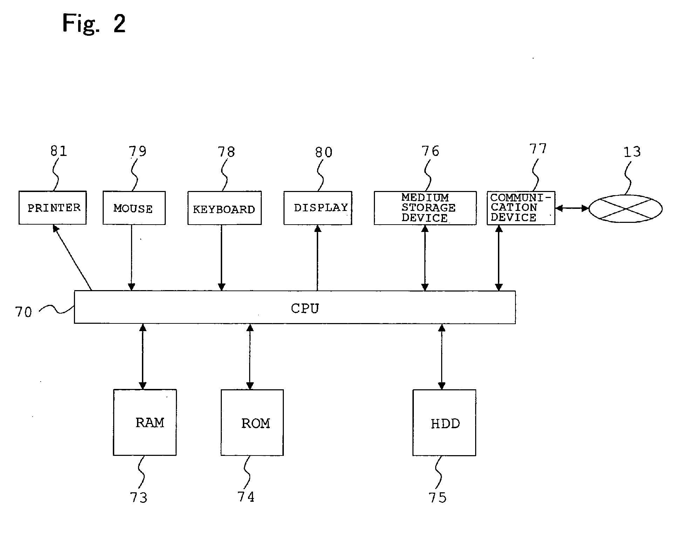

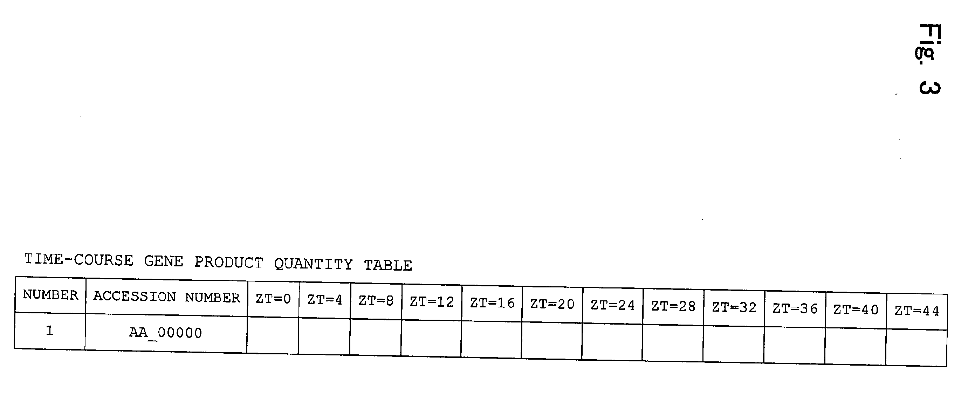

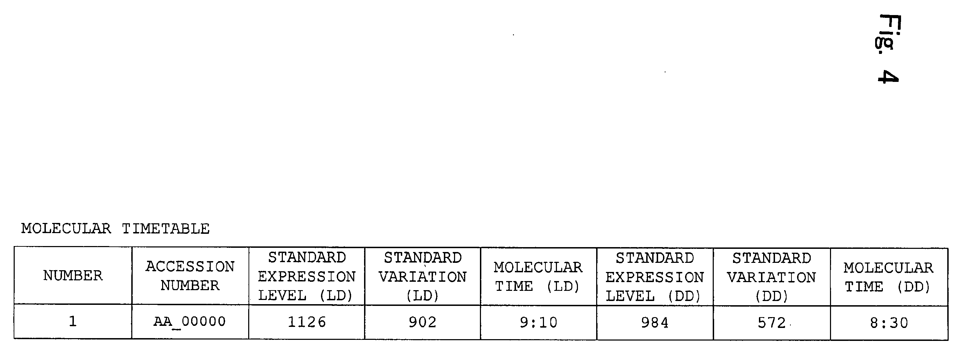

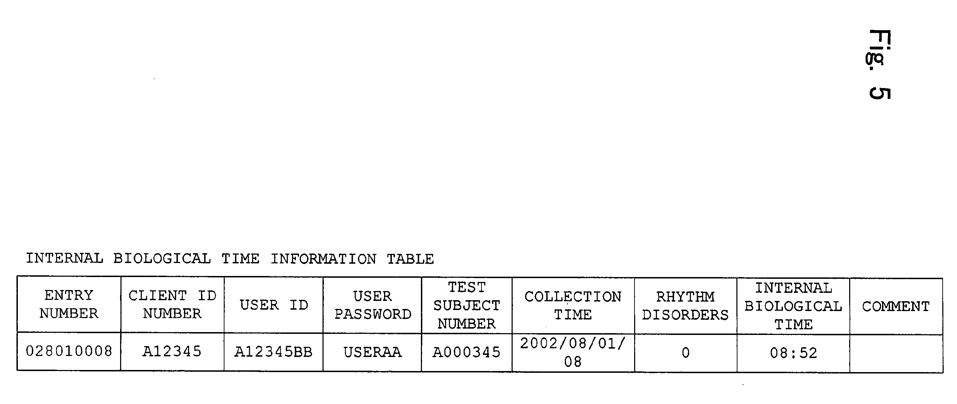

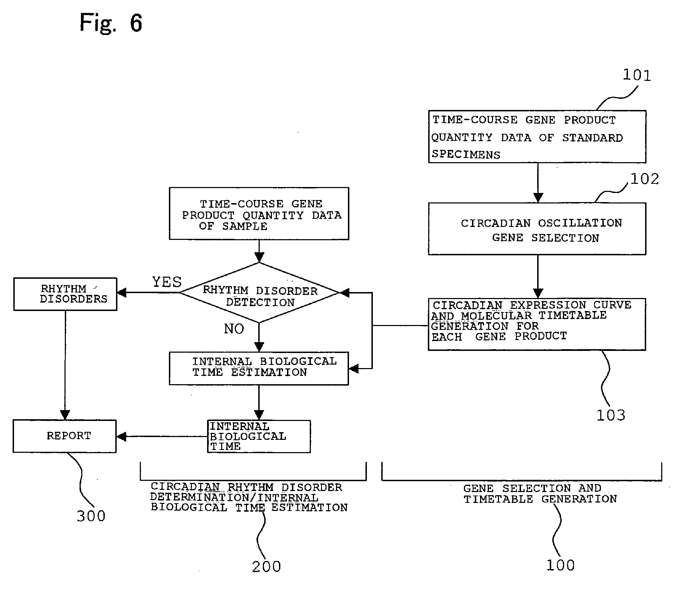

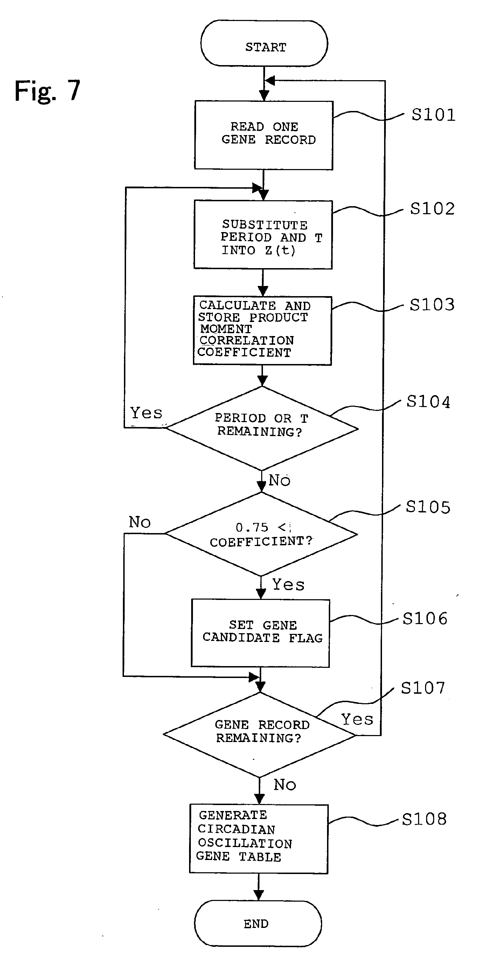

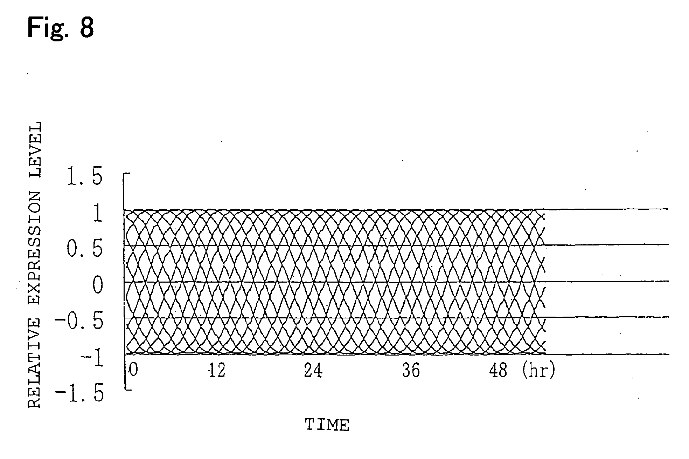

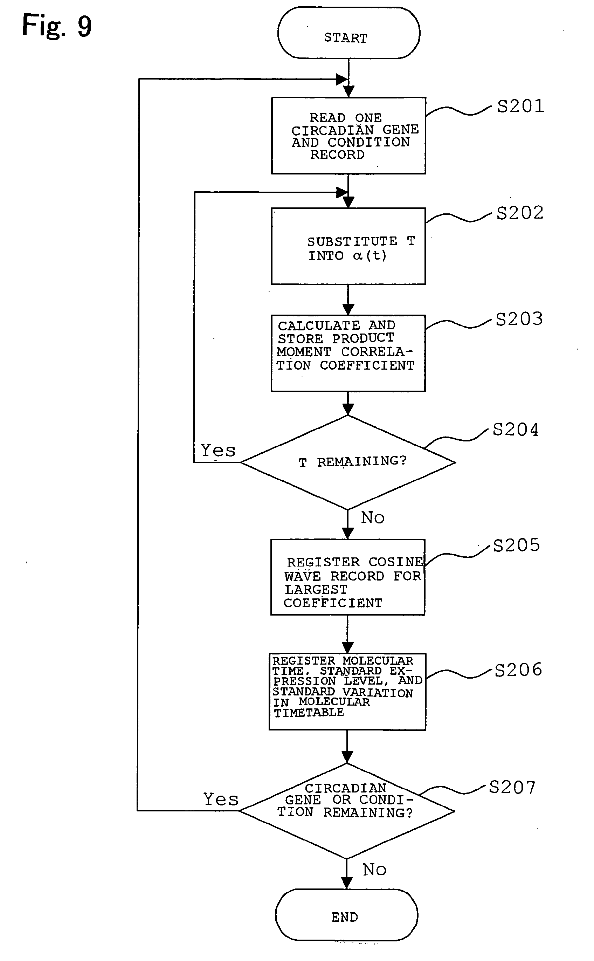

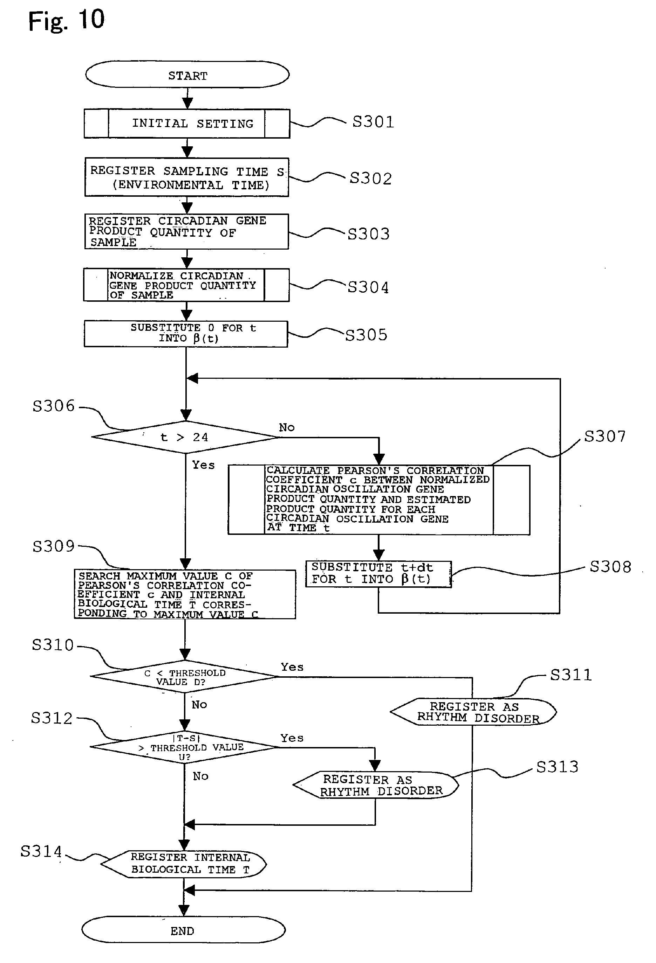

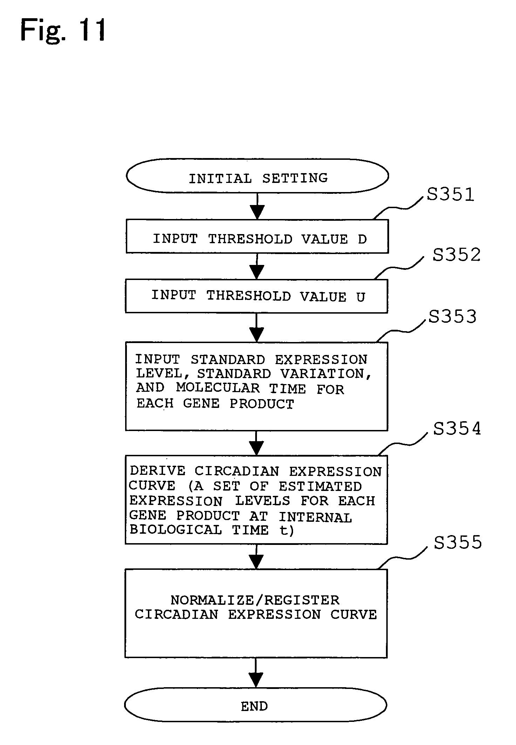

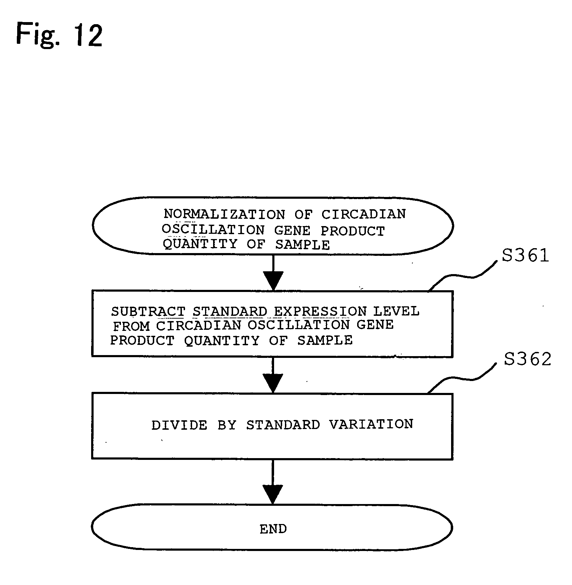

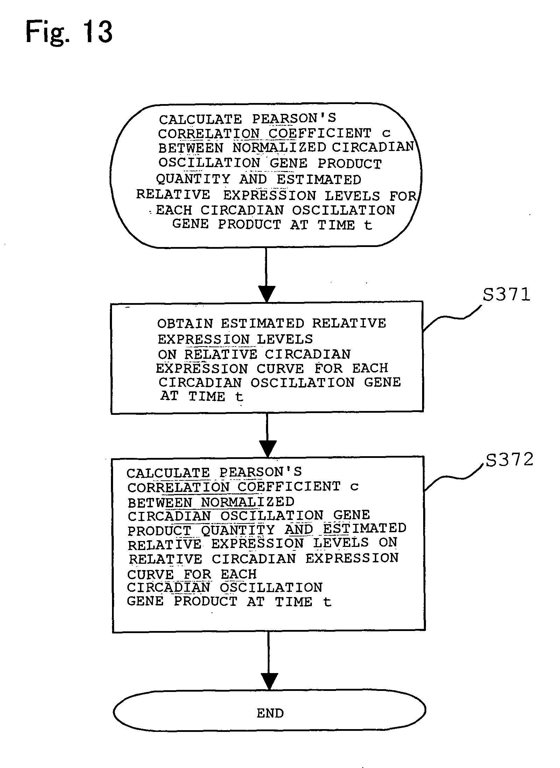

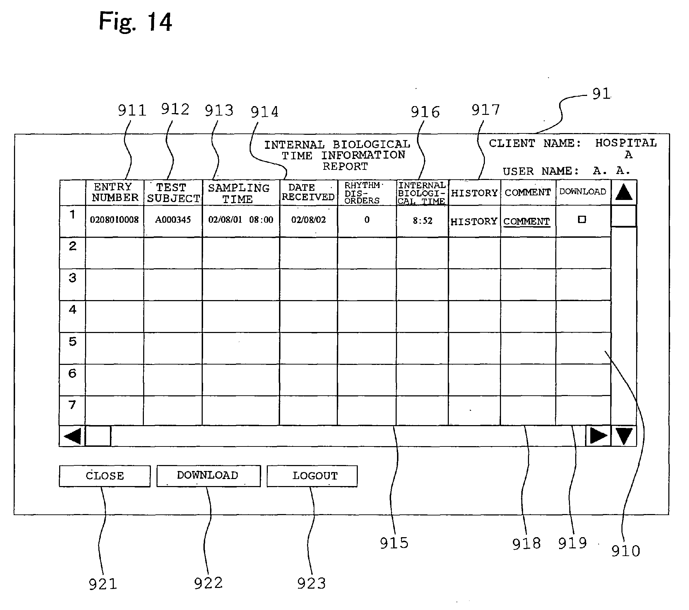

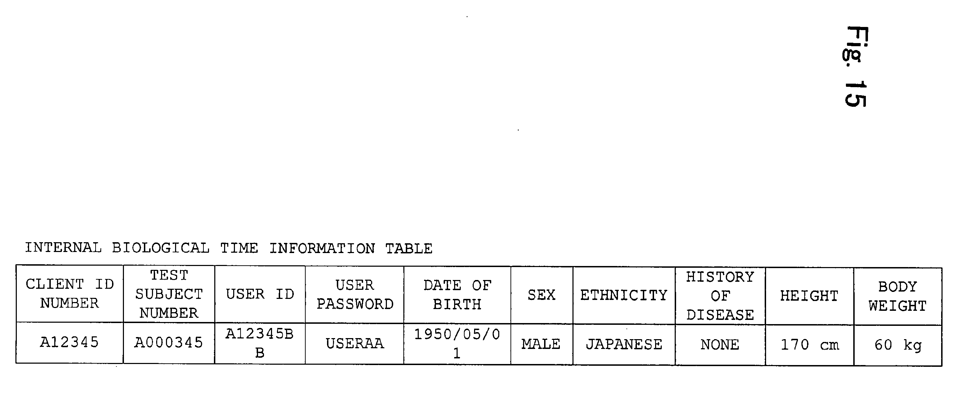

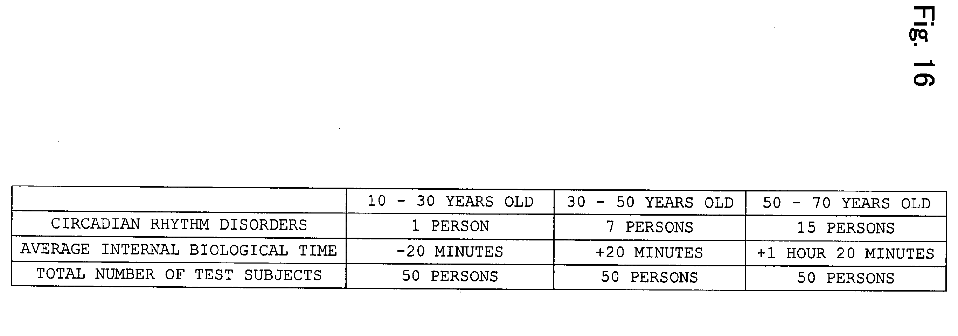

FIG. 1 is an explanatory diagram showing a general configuration of the internal biological time estimation system according to one embodiment of the present invention; FIG. 2 is an explanatory diagram showing a hardware configuration of the internal biological time estimation apparatus for general management of the internal biological time estimation system according to one embodiment of the present invention; FIG. 3 is an explanatory diagram showing the makeup of a time-course gene product quantity table; FIG. 4 is an explanatory diagram showing the makeup of a molecular timetable; FIG. 5 is an explanatory diagram showing the makeup of an internal biological time information table; FIG. 6 is a block diagram showing a process flow of the internal biological time estimation system according to one embodiment of the present invention; FIG. 7 is a flowchart showing the circadian oscillation gene selection process according to one embodiment of the present invention; FIG. 8 an explanatory diagram showing cosine waves for gene selection; FIG. 9 is a flowchart showing a process for creating circadian expression curves and molecular timetables according to one embodiment of the present invention; FIG. 10 is a flowchart showing an estimation process for internal biological time and information provision process according to one embodiment of the present invention; FIG. 11 is a flowchart showing an initial setting process, which is part of the estimation process for internal biological time and information provision process according to one embodiment of the present invention; FIG. 12 is a flowchart showing a normalization process for circadian oscillation gene product quantity of a sample, the normalization process being part of the estimation process for internal biological time and information provision process according to one embodiment of the present invention; FIG. 13 is a flowchart showing a calculation process of the Pearson's correlation coefficient c between a normalized circadian oscillation gene product quantity and an estimated expression level for each of circadian oscillation genes at time t, the calculation process being part of the estimation process for internal biological time and information provision process according to one embodiment of the present invention; FIG. 14 is an explanatory diagram showing an internal biological time information report screen according to one embodiment of the present invention; FIG. 15 is an explanatory diagram showing a test subject characteristics table; and FIG. 16 is an explanatory diagram showing exemplary data analysis results. Best Mode for Carrying Out the Invention

The embodiments of the present invention will next be described with reference to the drawings. The elements, configurations, etc. described hereinbelow should not be construed as limiting the present invention, and various modifications can be made within the scope of the invention.

The internal biological time estimating system of the present invention derives, from the gene product quantity data of a human specimen input in or received by the server computer in an information center, internal biological time information including a judgment as to whether or not the specimen donor suffers a circadian rhythm disorder, and in the case where the specimen donor is determined not to have a circadian rhythm disorder, an estimated internal biological time of the specimen donor, and then transmits the internal biological time information to a terminal computer of the client who requested the test.

According to the present embodiment, internal biological time of a human is estimated, but the internal biological time estimating system of the present embodiment may be employed for estimating internal biological time of any living organism so long as the organism has a circadian rhythm, and examples include mammals such as mice, rats, and dogs.

Also, the present embodiment will be described in relation to a case where internal biological time of an individual is estimated by use of a human blood specimen, but any specimen may be employed for estimating internal biological time, so long as the specimen is originating from an organism having a circadian rhythm. Examples of such a specimen include the suprachiasmatic nucleus or the liver of a mouse, drosophila heads, human skin, white blood cells from peripheral blood, mucous membrane of the oral cavity, and other organs.

Further, according to the present embodiment, quantity data for a gene transcription product, messenger RNA (hereinafter referred to as mRNA), are employed as the gene product quantity data, but any measurement data may be employed, so long as the data exhibit a circadian rhythm. Examples of the measurement data include those of the mass of a protein coded by a gene, those of modification levels of a protein coded by a gene, those of activity levels of an enzyme coded by a gene, those of a compound metabolized by an enzyme, and those related to physical items regulated by gene products, such as body temperature, blood pressure, heart rate, blood flow, respiration rate, brain waves, activity level, and pH of a body fluid.

The term "circadian oscillatory gene" denotes a gene which is oscillatorily expressed with a cycle period of about 24 hours (20 to 28 hours) under light/dark conditions (i.e., alternating 12-hour light period/12-hour dark period) or constant dark conditions.

The term "standard expression level" denotes a mean value of time-course expression level measurement data of a gene; "standard variation" denotes standard deviation of time-course expression level measurement data of the gene; and "relative expression level" denotes an expression level normalized by subtracting the standard expression level from a measurement value in items of a gene product of a gene, followed by division by the standard variation.

Normalization is means for assessing expression variation of each gene on the same basis, and specifically, the term "normalized" or "normalization" denotes a process of subtracting the standard expression level from the measured value for the quantity of a gene product of each gene, or a value extracted from a corresponding circadian expression curve, followed by division by the standard variation. Through this normalization process, the standard variation and the standard expression level of each circadian oscillatory gene become 1 and 0, respectively, resulting in unified treatment of these variables.

A "circadian expression curve" is drawn from a numerical formula of time-course expression profile of a circadian oscillatory gene derived from a finite number of expression data. The curve can be obtained through any of a plurality of methods on the basis of expression levels measured over time. A "relative circadian expression curve" is a normalized circadian expression curve obtained by subtracting the standard expression level from a value extracted form a corresponding circadian expression curve, followed by division by the standard variation.

A "ZT time" is a time measured from when a light is turned on (ZT0) under the light/dark conditions. The light remains on for 12 hours, and then the light is turned off at ZT12. Thus, the light period is from ZT0 to ZT12, and the dark period is from ZT12 to ZT24.

A "CT time" is a time determined on a subject which has undergone rhythm synchronization, or entrainment, to a 24-hour light/dark period. The beginning of the CT time, CT0, corresponds to ZT0, but under the CT timescale, the light is not turned on at CT0. The CT time is measured under constant dark conditions and has a period of 24 hours.

A "molecular time" is a time on a CT timescale (CT time) or a time on a ZT timescale (ZT time), at which the circadian expression curve of the circadian oscillatory gene exhibits the maximum value.

Relations between objects which are "similar" to one another are expressed by numerical values. Such relations include "similarity," which is used when, as in the case of correlation coefficient, a larger value represents a more similar relation, and "dissimilarity," which is used when, as in the case of distance, a smaller value represents a less similar relation.

The "distance," one of the indices representing dissimilarity, is an index showing how far the objects are separated from one another. The "squared Euclidian distance" is one of the most commonly used distance, and the distance $d_{xy}$ between variables x and y is defined as follows when the data are given in the form of $(x_1, y_1)$, $(x_2, y_2)$, $(x_3, y_3)$,..., $(x_n, y_n)$ (see Reference 3).

$$d_{xy} = \sum_{i=1}^{N} (x_i - y_i)^2 \qquad (1)$$

The "correlation coefficient," one of the indices representing similarity, shows the degree of correlation. The "Pearson's product moment correlation coefficient" is one of the most commonly used correlation coefficients and is defined as follows (see Reference 4).

$$\gamma_{xy} = \frac{\displaystyle\sum_{i=1}^{N} \frac{(x_i - \bar{x})(y_i - \bar{y})}{n}}{\sqrt{\displaystyle\sum_{i=1}^{N} \frac{(x_i - \bar{x})^2}{n}} \sqrt{\displaystyle\sum_{i=1}^{N} \frac{(y_i - \bar{y})^2}{n}}} \qquad (2)$$

where

$$\bar{x} \equiv \frac{\displaystyle\sum_{i=1}^{N} x_i}{n}, \ \bar{y} \equiv \frac{\displaystyle\sum_{i=1}^{N} y_i}{n} \qquad (3)$$

Next, the standard deviation s of a cosine wave will be described. The value A(t) of the cosine wave at time t can be obtained from:

$$A(t) = Cos\left(\frac{2\pi(t-T)}{Period}\right) \qquad (4)$$

where

Period: 24 hours,
T: the molecular time on the cosine wave (e.g., 144 kinds of times at 10-minute intervals; i.e., 0/60, 10/60, 20/60, 30/60, ..., 1420/60, and 1430/60), and
t: corresponding time.

The standard deviation s of the time-course data (a set of values at relevant sampling points in time) on the cosine wave can be obtained from the following formula.

$$s = \sqrt{\frac{n\displaystyle\sum_{i=1}^{n} (A(t)_i)^2 - \left(\displaystyle\sum_{i=1}^{n} (A(t))i\right)^2}{n(n-1)}} \qquad (5)$$

If the time-course data on the cosine wave are obtained at intervals of 4 hours, t can be expressed by the following set of the 6 time points (n=6): $(\alpha+0)/60$, $(\alpha+240)/60$, $(\alpha+480)/60$, $(\alpha+720)/60$, $(\alpha+960)/60$, and $(\alpha+1200)/60$, where $\alpha$ is a certain time between 0 and 240 minutes (exclusive of 240 minutes). In this case, the standard deviation is 0.7745967.
In the present specification, the term "computer" is used to mean any information terminal equipped with an arithmetic unit. Examples of the computers include a supercomputer, a general purpose computer, an office computer, a computer for control purposes, a workstation, and a personal computer, and further include a cellular information terminal, a cellular phone equipped with an arithmetic unit, and a wearable computer.

Also, as used herein, the term "input" means inputting an instruction or data to a computer, and includes inputting an instruction or data through a keyboard or a mouse, reading an instruction or data from a storage medium such as a flexible disk or a compact disk, and receiving an instruction or data from an external unit through a communication line.

(Embodiment 1)

As shown in FIG. 1, the internal biological time estimating system according to this embodiment of the present invention includes an information center 1 providing internal biological time information of an individual to a client and clients such as a medical facility 2, a sports center 3, an individual member 4, a health care facility for the elderly 5, a corporate member 6, and an educational institution 7. These clients receives, from the information center 1, internal biological time information of an individual.

The information center 1 has a server computer 11, and terminal computers 21 to 71 belonging to clients 2 to 7 are connected thereto via the Internet 13.

The information center 1 is a general administration organization for operating the internal biological time estimating system of the present embodiment, and may be included in a clinical test company, a pharmaceutical company, a medical facility, a research institute, an analysis center, an examination center, a data center, or the like.

The information center 1 includes a DNA chip reader 12 for measuring the quantity of a gene product of a specimen and inputting the gene product quantity data to the server computer 11; and the server computer 11, serving as an internal biological time information providing unit, for performing, by use of the gene product quantity data transmitted from the DNA chip reader 12, processes related to judgment on the presence or absence of a circadian rhythm disorder or to estimation of internal biological time, and subsequently transmitting to any of the terminal computers 21 to 71 the internal biological time information obtained through the processes.

In the present embodiment, the gene product quantity data are sent from the DNA chip reader 12. Alternatively, an apparatus for measuring the gene product quantity may be installed in each of the clients 2 to 7 for sending the data from the terminal computers 21 to 71.

The information center 1 receives, from the clients 2 to 7, by mail or walk-in, blood samples 19 drawn from subjects undergoing clinical tests (hereinafter may be referred to as simply test subjects or subjects). Each blood sample 19 is pretreated through a known method, to thereby make the sample ready for measurement of expressed mRNA level of a gene. For example, in one known method, mRNA is separated/purified from the blood sample 19 by use of a commercial RNA extraction kit (e.g., RNAqueous-Blood Module, RNAqueous Phenol-free Total RNA kit, Funakoshi Co., Ltd.) with reference to the protocol provided by the manufacturer. Purification of the blood sample 19 may be performed by extracting mRNA by use of, for example, the guanidine thiocyanate/cesium chloride method, the guanidine thiocyanate hot phenol method, or the guanidine thiocyanate - guanidine hydrochloride method, and then adsorbing/eluting mRNA on an oligo(dT)-cellulose column.

Subsequently, the quantity of expressed mRNA in the treated blood sample 19 is measured by the DNA chip reader 12. The thus-obtained raw mRNA quantity data are accumulated in the DNA chip reader 12.

The raw data accumulated in the DNA chip reader 12 are then transmitted to the server computer 11. The server computer 11 processes the raw data, determines whether the relevant test subject has circadian rhythm disorders, and, if the subject does not have circadian rhythm disorders, calculates the internal biological time of the subject.

The results of judgment as to whether or not the test subject has a circadian rhythm disorder and, in the case where the subject does not have a circadian rhythm disorder, the calculated internal biological time of the test subject are transmitted and reported, as the biological time information of the test subject, to the relevant terminal computer; i.e., the relevant one of the terminal computers 21 to 71, belonging to the client who requested the test.

The clients 2 to 7 are clients of the information center 1 and receive the internal biological time information produced by the internal biological time estimating system of the present embodiment at the information center 1.

Each of the clients 2 to 7 has a terminal computer; i.e., a relevant one of the terminal computers 21 to 71, which enables the client to view the display internal biological time information display screen provided by the server computer 1 at the information center 1.

A gene product quantity measuring apparatus for measuring the quantity of a gene product may be installed in each of the clients 2 to 7. In such a case, the expression level of mRNA of a blood sample 19 of a test subject is measured at any of the clients 2 to 7, and the obtained data are transmitted to the server computer 11 from the corresponding one of the terminal computers 21 to 71, and subsequently the same one of the terminal computers 21 to 71 receives, from the server computer 11, the judgment as to whether or not the data indicate the presence of a circadian rhythm disorder and the internal biological time information derived from the data.

The medical facility 2 utilizes the internal biological time estimating system of the present embodiment for purposes of, among other purposes, determining whether or not there is involved a circadian rhythm disorders in a patient suffering a sleep-wake rhythm disorder, seasonal depression, or jet-time syndrome or, in the case where the patient is a day-night shift worker, suffering a sleep disorder, or determining the time at which a patient should take a medicine.

The medical facility 2 has an electronic medical record server computer 22 for storing electronic data on medical treatment records, prescriptions, clinical test results, and other information pertaining to individual patients. The electronic medical record server computer 22 is connected to a terminal computer 21 via an intra-hospital LAN, and is configured such that internal biological time information downloaded from the server computer 11 to the terminal computer 21 can be input to the electronic medical record server computer 22.

The electronic medical record server computer 22 may be configured such that internal biological time information pertaining to a patient can be automatically and directly incorporated to his/her electronic medical record. This can be realized by allotting to each patient, as his/her test subject number (described hereinbelow), his/her patient ID number registered in the electronic medical record server computer 22, and by installing a program which automatically transfers, to a medical record database (not shown) provided in the electronic medical record server computer 22, the internal biological time information pertaining to each test subject downloaded from the server computer 11 to the terminal computer 21.

The medical facility 2 may be replaced by, for example, a psychological counselor, a facility for health and welfare such as a health center, a clinic, a child counseling facility, or a pharmacy.

The sports center 3 is an organization in which athletes are coached, trained, or nurtured. No particular limitation is imposed on the types of the sports. In the sports center 3, the internal biological time estimating system of the present embodiment is used to establish training schedules for athletes who are scheduled to take part in international sports events.

The sports center 3 has, in addition to a terminal computer 31, an athlete management server computer 32 which stores electronic data, for each athlete, concerning, for example, health conditions, a training menu, a diet menu, or event participation records pertaining to an athlete. The athlete management server computer 32 is connected to the terminal computer 31 via an intra-center LAN, and is configured such that internal biological time information downloaded from the server computer 11 to the terminal computer 31 can be input to the athlete management server computer 32.

The athlete management server computer 32 may be configured such that internal biological time information pertaining to each athlete can be automatically and directly incorporated to a database (not shown) provided in the athlete management server computer 32. This can be realized by allotting to each athlete, as his/her test subject number (described hereinbelow), his/her athlete ID number registered in the athlete management server computer 32, and by installing a program which automatically transfers, to the unillustrated database, the internal biological time information pertaining to the athlete downloaded from the server computer 11 to the terminal computer 31.

The sports center 3 may be replaced by a sports gym, a sports club, a legal person who manages club activities, or an educational institution which governs club activities.

The individual member 4 is a client who privately utilizes the internal biological time estimating system of the present embodiment for the purpose of improving his/her own life.

Examples of the individual member 4 include a health-conscious individual, a senior citizen, a person who has experienced to suffer a sleep-wake rhythm disorder, seasonal depression, jet-lag syndrome, or a similar disorder, and a day-night shift worker. When a family member of an individual member 4 or a person who cares for an individual member 4 can draw a specimen from the individual member, the family member or the person, instead of the individual member 4 himself/herself, may draw a specimen and view or handle the internal biological time information of that individual.

The health care facility for the elderly 5 makes use of the internal biological time estimating system of the present embodiment for purposes of health management and daily life management of the elderly staying in the facility.

For example, internal biological time information may be used as information for allotting rooms to potential inmates.

When an aged person moves in such a facility, the facility acquires his or her internal biological time information from the information center 1, whereby inmates having similar internal biological time patterns can be grouped to share a room. This facilitates administration and management over inmates (e.g., patrol at wake-up time and at bedtime), as the inmates in the same room have similar internal biological time and thus can live on similar behavioral paces.

In addition, if internal biological time information of an aged person is acquired at the time when he or she moves in the facility, the staff of the facility can adequately respond to his or her daily life rhythm from an early stage after his or her relocation, facilitating adaptation of the new comer to the general timetable of the facility from an early time.

The health care facility for the elderly 5 has, in addition to a terminal computer 51, a management server computer 52 which stores electronic data, for each inmate, concerning health conditions, a diet menu, information about family members, a rehabilitation menu, or other information pertaining to an inmate. The management server computer 52 is connected to the terminal computer 51 via an intra-facility LAN, and is configured such that internal biological time information downloaded from the server computer 11 to the terminal computer 51 can be input into the management server computer 52.

The management server computer 52 may be configured such that internal biological time information pertaining to each inmate can be automatically and directly incorporated to a database (not shown) provided in the management server computer 52. This can be realized by allotting to each inmate, as his/her test subject number (described hereinbelow), his/her inmate ID number registered in the management server computer 52, and by installing a program which automatically transfers, to the unillustrated database, the internal biological time information pertaining to the inmate downloaded from the server computer 11 to the terminal computer 51.

Other than the health care facility for the elderly 5, example clients include hospitals for the elderly, senior welfare facilities, nursing homes, and companies providing services for supporting family members who care for aged persons.

The corporate member 6 utilizes the internal biological time estimating system of the present embodiment for purposes of, for example, management of employees' health conditions and improvement of employees' work performance.

The corporate member 6 has, in addition to a terminal computer 61, an employee information server computer 62 which stores electronic data, for each employee, concerning, for example, working hours, working time zone, jobs or tasks, health conditions, and the age of the employee. The employee information server computer 62 is connected to the terminal computer 61 via a LAN, and is configured such that internal biological time information downloaded from the server computer 11 to the terminal computer 61 can be input to the employee information server computer 62.

The employee information server computer 62 may be configured such that internal biological time information pertaining to each employee can be automatically and directly incorporated to the employee information. This can be realized by allotting to each employee, as his/her test subject number (described hereinbelow), his/her employee ID number registered in the employee information server computer 62, and by installing a program which automatically transfers, to an unillustrated database, the internal biological time information pertaining to the employee downloaded from the server computer 11 to the terminal computer 61.

The corporate member 6 may be replaced by, for example, a public agency or a domestic or abroad branch office thereof.

The educational institution 7 utilizes the internal biological time estimating system of the present embodiment for purposes of, among other purposes, management of physical or mental health of pupils and students or improvement of education efficiency.

The educational institution 7 has, in addition to a terminal computer 71, a pupil or student information server computer 72 which stores electronic data concerning, for example, academic records, home environment, health conditions, or the age of each pupil or student. The pupil or student information server computer 72 is connected to the terminal computer 71 via a LAN, and is configured such that internal biological time information downloaded from the server computer 11 to the terminal computer 71 can be input to the pupil or student information server computer 72.

The pupil or student information server computer 72 may be configured such that internal biological time information pertaining to each pupil or student can be automatically and directly incorporated to information pertaining to each pupil or student. This can be realized by allotting to each pupil or student, as his/her test subject number (described hereinbelow), his/her pupil or student ID number registered in the pupil or student information server computer 72, and by installing a program which automatically transfers, to an unillustrated database provided in the pupil or student information server computer 72, the internal biological time information pertaining to each pupil or student downloaded from the server computer 11 to the terminal computer 71.

The educational institution 7 may be replaced by a research organization such as a university or a laboratory.

The clients 2 to 7 collect blood samples from test subjects and place the samples in test tubes provided by the information center 1. The samples will hereafter be referred to as blood samples 19. At the time when the blood samples are placed in the test tubes, each test tube is labeled with a client ID number given uniquely to each client, a test subject number identifying the test subject, and year/month/day and time at which the sample was drawn. The clients 2 to 7 submit the blood samples 19 to the information center 1 by mail or walk-in.

One or two days after submission of the blood samples 19, the clients 2 to 7 can view, on terminal computers 21 to 71, the judgment results as to whether or not the individual test subjects suffer circadian rhythm disorders and, in the case of the absence of circadian rhythm disorders, internal biological times of respective test subjects.

When the clients 2 to 7 each have a gene product quantity measuring apparatus, the clients 2 to 7 can measure on their side mRNA expression levels of the blood samples 19 and transfer, from the terminal computers 21 to 71 to the server computer 11, the relevant measurement data together with information including the corresponding client ID numbers which have been given in advance, the corresponding test subject numbers, and the year/month/day and time at which the samples were drawn.

Within several hours from transfer of the measurement data, the clients 2 to 7 can view, on terminal computers 21 to 71, the judgment results as to whether or not the individual test subjects suffer circadian rhythm disorders and, in the case of the absence of circadian rhythm disorders, internal biological times of respective test subjects.

The apparatuses and computer will next be described.

A DNA chip reader 12 may be a known DNA chip reader and includes a DNA chip scanner (not shown), a CPU (not shown) for processing the data read by the scanner, and a storage for storing the processed data.

The DNA chip reader 12 measures the amounts of respective mRNA species among the total RNA contained in a specimen, and the measurement data are stored in the storage (not shown).

Although the present embodiment employs a DNA chip reader 12 to measure the quantity of a gene product, there may be employed any other suitable apparatuses for, for example, quantitative PCR, real-time PCR, DNA microarray assays, RNase protection assays, or northern hybridization assays.

When a protein level measurement is desired to be used as the quantity of a gene product, there may be employed a known apparatus capable of performing, for example, two-dimensional electrophoresis, mass spectroscopy, protein chip analysis, antibody chip analysis, or immunoblotting.

The server computer 11 determines, by use of the gene product quantity data, whether or not the specimen indicates circadian rhythm disorders and estimates the internal biological time of the test subject related to the specimen.

The server computer 11, as shown in FIG. 2, is equipped with a central processing unit (CPU 70), storage devices (RAM 73, ROM 74, HDD 75, and a storage medium device 76), a communication device 77, a keyboard 78, a mouse 79, a display 80, and a printer 81.

The CPU 70 performs calculations by use of the information from the storage devices, the communication device, and the input device, and plays a role to transmit the calculation results to the storage devices, the communication device, and the output device.

The storage devices store programs and the like to perform various processes. Among the storage devices, the storage medium device 76 includes external medium devices such as HDD, CD, and DVD, and, as occasion requires, can store or read out data received via the communication device 77 or data input through the keyboard 78 or the mouse 79.

The RAM 73 stores data, etc. which are necessary for the CPU 70 to perform processes. The display 80 displays the image or the like which the CPU 70 created with programs stored in the ROM 74, HDD 75, or the like. The printer 81 outputs predetermined items of information from respective computers on paper. The communication device 77 transmits/receives data to and from other computers including the DNA chip reader 12 and the terminal computers 21 to 71.

The HDD 75 contains the following items: a time-course gene product quantity table 14 as shown in FIG. 3; a circadian oscillatory gene table (not shown) including circadian oscillatory gene candidates' time-course gene product quantity data chosen from standard specimen's time-course gene product quantity data; a molecular timetable 15 as shown in FIG. 4, an internal biological time information table 16 as shown in FIG. 5; cosine wave information for selecting genes (not shown), and cosine wave information for generating timetables (not shown).

The time-course gene product quantity table 14, as shown in FIG. 3, is a table where the time-course data regarding the quantity of a gene product contained in a standard specimen is registered for each gene.

The molecular timetable 15, as shown in FIG. 4, is a table to be used to determine whether or not the sample shows the presence of a circadian rhythm disorder and to estimate internal biological time through the estimating process. The molecular timetable is formed of numerical value items that characterize the circadian expression curve of each of the circadian oscillatory genes chosen from a standard specimen; i.e., standard expression level 153, standard variation 154, and molecular time 155, all these three items being determined under LD conditions (24-hour cyclic light/dark conditions with a 12-hour light period and a 12-hour dark period), as well as standard expression level 156, standard variation 157, and molecular time 158, all these three items being determined under DD conditions (constant dark conditions).

The internal biological time information table 16, as shown in FIG. 5, is a table where the judgment concerning whether or not the sample from a test subject shows a circadian rhythm disorder and the results from the estimation process for determining the internal biological time of the sample from the test subject are tabulated as records. The respective records are registered when the process for estimating internal biological time and providing information, as shown in the flowchart of FIG. 10, is executed.

The internal biological time information table 16 is formed of the following registration items: entry number 161 allotted to each specimen by the information center 1 upon receipt of a blood sample 19, client number 162 allotted to each client at the time of the client's membership registration, user ID 163 of a person in charge at the client, user password 164 of the person in charge, test subject number 165 allotted to each test subject on the client's side, sample collection time 166 which is indicated by way of labeling on the test tube of the blood sample 19 at the time of collection of the blood sample 19 on the client's side and which is input to a computer at the information center 1, judgment as to the presence or absence of circadian rhythm disorders 167 rendered by the server computer 11, internal biological time estimation results 168 rendered by the server computer 11, and comments 169 entered as needed in relation to each specimen on the information center 1's side.

The cosine wave information for selecting genes is information in the form of cosine waves which are used for selecting circadian oscillatory genes selected from among the genes contained in a standard specimen.

The number of the cosine waves for gene selection is 540; i.e., there are 540 cosine waves having different periods of 20 to 28 hours with increments of 1 hour and with phases shifted by one sixtieth the period. That is, for each of the 9 periods, 60 cosine waves are drawn with their phases shifted. A portion of these cosine waves to be used for gene selection are shown in FIG. 8.

The cosine wave information for gene selection comprises the following formula A(t) used for calculating a value z(t) on a cosine wave for gene selection:

$$A(t) = Cos\left(\frac{2\pi(t-T)}{Period}\right) \qquad (6)$$

where

period: 9 different hours; i.e., 20, 21, 22, 23, 24, 25, 26, 27, and 28
T: each point in time where a period is divided into 60 equal portions and where the cosine wave reaches the maximum value t: a certain point in time

The cosine wave information for generating timetables is information in the form of cosine waves which are used for generating molecular timetables of circadian oscillatory genes selected from among the genes contained in a standard specimen.

The number of the cosine waves for timetable generation is 144; i.e., there are 144 cosine waves (period of each wave: 24 hours) having phases shifted by 10 minutes.

The cosine wave information for timetable generation comprises the following formula A(t) used for calculating a value $\alpha$ (t) on a cosine wave for timetable generation:

$$A(t) = Cos\left(\frac{2\pi(t-T)}{Period}\right) \qquad (7)$$

where

period: 24 (hours)
T: molecular time on the cosine wave (for example, 144 different points in time with intervals of 10 minutes (0/60, 10/60, 20/60, 30/60, ..., 1420/60, and 1430/60))
t: a certain point in time

Next, based on FIG. 6, the process flow of the internal biological time estimating system of the present embodiment will be described.

Information center 1 provides, at the server computer 11, internal biological time information access services for clients via Internet 13.

As shown in FIG. 6, the process performed by the internal biological time estimating system generally includes a gene selection/timetable generation stage 100, a circadian rhythm disorder judgment/internal biological time estimation stage 200, and a reporting-to-clients stage 300.

The gene selection/timetable generation stage 100 is a preparatory stage for the circadian rhythm disorder judgment/internal biological time estimation stage, and is performed before an internal biological time information provision service is available to a client.

The portion—or in other words—type of the tissue/cells used in circadian rhythm disorder judgment/internal biological time estimation and a circadian oscillatory gene are selected, and then a molecular timetable of the circadian oscillatory gene is generated.

In the gene selection/timetable generation stage 100, firstly, time-course gene expression data of standard specimens are obtained in step 101. This data acquisition is carried out by use of a DNA chip reader 12.

The standard specimens are biological tissues sampled from a plurality of biological individuals. In the present embodiment, the standard specimens are blood drawn from a plurality of humans.

As the standard specimens, blood is sampled at predetermined time intervals from a plurality of humans under predetermined conditions. Specifically, after a plurality of examinees are forced to stay in a room under a 24-hour cycle of 12-hour-light and 12-hour-dark conditions for 2 weeks, blood is drawn every 4 hours over 2 days under the same 24-hour cycle of 12-hour-light and 12-hour-dark conditions (LD conditions) or under constant dark conditions (DD conditions).

Blood sampling under LD conditions is performed at the following 12 points in time: immediately before lights on (ZT0), 4 hours after lights on (ZT4), 8 hours after lights on (ZT8), immediately before lights off (ZT12), 4 hours after lights off (ZT16), 8 hours after lights off (ZT20), and on Day 2, immediately before lights on (ZT24), on Day 2, 4 hours after lights on (ZT28), on Day 2, 8 hours after lights on (ZT32), on Day 2, immediately before lights off (ZT36), on Day 2, 4 hours after lights off (ZT40), and on Day 2, 8 hours after lights off (ZT44). Blood sampling under DD conditions are the following 12 points in time: immediately before the start of DD conditions (CT0), 4 hours after the start of subjective daytime (CT4), 8 hours after the start of subjective daytime (CT8), immediately before the start of subjective night (CT12), 4 hours after the start of subjective night (CT16), 8 hours after the start of subjective night (CT20), and on Day 2, immediately before the start of subjective daytime (CT24), on Day 2, 4 hours after the start of subjective daytime (CT28), on Day 2, 8 hours after the start of subjective daytime (CT32), on Day 2, immediately before the start of subjective night (CT36), on Day 2, 4 hours after the start of subjective night (CT40), and on Day 2, 8 hours after start of subjective night (CT44).

Blood samples drawn from a plurality of humans are treated with known methods to thereby prepare, for each individual, each of the LD/DD conditions, and each sampling time, a standard specimen for measuring the mRNA quantity, which is a gene product quantity.

The mRNA quantity of a standard specimen—prepared for each individual, each of the LD/DD conditions, and each sampling time—is measured by use of a DNA chip reader 12, whereby time-course gene product quantity data of respective standard specimens are obtained.

The obtained time-course gene product quantity data are stored within a storage device (not shown) of the DNA chip reader 12, and transmitted therefrom to a server computer 11.

Next, in step 102, on the basis of the time-course gene product quantity data received in step 101, the server computer 11 selects circadian oscillatory genes.

The procedure for selecting circadian oscillatory genes is described in detail with reference to the circadian oscillatory gene selection flowchart shown in FIG. 7. The procedure of FIG. 7 is controlled by a CPU 70 of the server computer 11.

The server computer 11 receives time-course gene product quantity data from the DNA chip reader 12, and stores the data as a time-course gene product quantity table 14 on HDD 75. FIG. 3 shows the time-course gene product quantity table 14.

In the time-course gene product quantity table 14, the mRNA quantity which is associated with the expression level of a gene is registered at each point in time for each gene.

When the time-course gene product quantity table 14 is registered on HDD 75, the process starts following the flowchart shown in FIG. 7. Firstly, in step S101, one gene record item is read out from the time-course gene product quantity table 14.

Subsequently, in Step S102, one value for period and one value for T are substituted into the following formula z(t) which is derived by applying the standard variation and standard expression level of each gene to formula A(t) of the cosine wave information for gene selection acquired from HDD 75, to thereby specify one cosine wave from among 540 cosine waves.

$$z(t) = \frac{A}{B} Cos\left(\frac{2\pi(t-T)}{Period}\right) + C \qquad (8)$$

where

Period: 9 different hours; i.e., 20, 21, 22, 23, 24, 25, 26, 27, and 28
A: standard variation of gene A
B: standard deviation of cosine wave calculated from formulas (4) and (5) given hereinabove
C: standard expression level of gene A

T: when the period is divided into 60 equal portions, the point in time at which the cosine wave shows the maximum value,

t: a certain point in time.

Next, in step S103, a Pearson's product moment correlation coefficient is calculated between each of the values as determined at respective points in time of the gene record read in step S101 and each value obtained by substituting a corresponding 4-hour-interval point in time for t of formula z(t), and the calculation results are stored in RAM 73.

That is, a Pearson's product moment correlation coefficient is calculated between the value xn at time 4n in the gene record (i.e., the expression level at ZT4n or the expression level at CT4n, where n is an integer of 0 or more and 11 or less) and the value yn on a cosine wave at time 4n (i.e., the value on the cosine wave at ZT4n or the value on the cosine wave at CT4n, where n is an integer 0 or more and 11 or less) is calculated.

Next, in step S104, judgment is made as to whether or not there remains any period or T for which product moment correlation coefficient has not been calculated.

In the case where there remain periods and T for which product moment correlation coefficient has not been calculated (step S104: YES), in step S102, one value for period and one value for T are substituted into formula z(t) to thereby specify one cosine wave from among 540 cosine waves.

If there remains no period or T for which product moment correlation coefficient has not been calculated (step S104: NO), calculation and storage of product moment correlation coefficients are considered to have been completed for all the cosine waves for selection of genes, and in step S105, a determination is made as to whether or not there is any coefficient that is greater than 0.75 among all the product moment correlation coefficients that were calculated.

If there is found a coefficient value that is greater than 0.75 (step S105: YES), since the relevant gene is regarded as having an expression pattern oscillating with a period of between 20 to 28 hours inclusive and thus exhibiting a characteristic of the circadian oscillatory gene, in step S106, a gene candidate flag is set on its gene record.

Next, step S107 decides on whether or not there remains any gene record that has not yet undergone the process for calculating product moment correlation coefficient with cosine wave for gene selection. The product moment correlation coefficient calculation process is performed for each gene for both gene records; i.e., gene records under LD conditions and DD conditions.

If there remains a gene record that has not yet undergone the correlation coefficient calculation process (step S107: YES), in step S101, one gene record is read.

In the case where there remains no gene record that has not undergone the correlation coefficient calculation process (step S107: NO), all the genes are regarded as having undergone the judgment process for determining whether or not they are circadian oscillatory genes, and in step S108, an unillustrated circadian oscillatory gene table having circadian oscillatory gene candidates is generated. In step S108, referring to the time-course gene product quantity table 14, only such gene records that have gene candidate flags for both LD and DD conditions are extracted, to thereby generate an unillustrated circadian oscillatory gene table. The process is then terminated.

With the process described above, selection for circadian oscillatory genes in process 102 in FIG. 6 is completed.

Upon completion of the circadian oscillatory gene selection in process 102 in FIG. 6, in process 103, the server computer 11 generates a circadian expression curve and a molecular timetable for each gene product.

This process is described with reference to the circadian expression curve and molecular timetable generation flowchart shown in FIG. 9. The process of FIG. 9 is controlled by the CPU 70 of the server computer 11.

Once the selection of circadian oscillatory genes is completed in process 102 in FIG. 6, the flowchart of FIG. 9 starts.

Firstly, in step S201, for each of the LD and DD conditions, one circadian oscillatory gene record is read out from the unillustrated circadian oscillatory gene table produced in step S108 in FIG. 7.

Next, in step S202, one value for T is substituted into the following formula α(t) which is derived by applying the standard variation and standard expression level of each gene to formula A(t) of the cosine wave information for timetable generation acquired from HDD 75, to thereby specify one cosine wave from among 144 cosine waves.

$$\alpha(t) = \frac{A}{B} Cos\left(\frac{2\pi(t-T)}{24}\right) + C \qquad (9)$$

A: standard variation of circadian oscillatory gene A

B: standard derivation of cosine wave calculated from formulas (4) and (5) given hereinabove

C: standard expression level of gene A

T: molecular time on cosine wave (for example, the time T may be chosen from 144 points in time; i.e., 0/60, 10/60, 20/60, 30/60, ..., 1420/60, and 1430/60, in increments of 10 minutes), and

t: a certain point in time.

Next, in step S203, a Pearson's product moment correlation coefficient is calculated between each of the values as determined at respective points in time of the circadian oscillatory gene record read in step S201 and each value obtained by substituting a corresponding 4-hour-interval point in time for t of formula $\alpha(t)$, and the calculation results are stored in RAM73.

That is, a Pearson's product moment correlation coefficient is calculated between the value xn at time 4n in the circadian oscillatory gene record (i.e., the expression level at ZT4n or the expression level at CT4n, where n is an integer of 0 or more and 11 or less) and the value yn on a cosine wave at time 4n (i.e., the value on the cosine wave at ZT4n or the value on the cosine wave at CT4n, where n is an integer 0 or more and 11 or less) is calculated.

Next, in step S204, judgment is made as to whether or not there remains any T for which product moment correlation coefficient has not been calculated.

In the case where there remain T for which product moment correlation coefficient has not been calculated (step S204: YES), in step S202, one T value is substituted into formula z(t) to thereby specify one cosine wave from among 144 cosine waves.

If there remains no T for which product moment correlation coefficient has not been calculated (step S204: NO), calculation and storage of product moment correlation coefficients are considered to have been completed for all the cosine waves for timetable generation with regard to the circadian oscillatory gene record under the relevant LD/DD conditions, and in step S205, the cosine wave which provides the maximum value among all the product moment correlation coefficients calculated for the circadian oscillatory gene record under the relevant LD/DD conditions is extracted and stored in RAM73. This cosine wave is a circadian expression curve exhibiting an oscillatory expression pattern of the circadian oscillatory gene.

Subsequently, in step S206, the point in time at which the value on the circadian expression curve, which is extracted from the cosine waves, exhibits the maximum is registered in the molecular timetable 15 shown in FIG. 4 along with the name of the circadian oscillatory gene, the conditions (LD or DD), the standard expression levels, and the standard variations.

The standard expression levels 153 and 156, the standard variations 154 and 157, and the molecular times 155 and 158 characterize the circadian expression curve.

Subsequently, in step S207, a determination is made as to whether or not there remain any items of a circadian oscillatory gene record which has not undergone processing for calculation of a product moment correlation coefficient with the cosine wave for generating a timetable.

If there remain any items of a circadian oscillatory gene record which has not undergone processing for calculation of a product moment correlation coefficient with the cosine wave for generating a timetable (step S207: YES), in step S201, one circadian oscillatory gene record is read, for each of LD and DD conditions, from a circadian oscillatory gene table (not shown) generated in step S108 in FIG. 7.

If there remain no items of a circadian oscillatory gene record which has not undergone processing for calculation of a product moment correlation coefficient with the cosine wave for generating a timetable (step S207: NO), procedures for generating circadian expression curves and molecular timetables are considered to have been completed for all the circadian oscillatory genes, and the process is terminated.

Through the above steps, the process 103 in FIG. 6 for generating circadian expression curves and molecular timetables for respective genes is completed.

Thus, the gene-selection/timetable-generation stage 100 is completed, and services for providing internal biological time information to clients are now available.

Upon completion of the gene-selection/timetable-generation stage 100, the information center 1 starts services for providing internal biological time information.

Users at clients 2 to 7 who are desirous of using the internal biological time estimating system of the present embodiment obtain a membership status with the information center 1 in advance.

Membership registration is done by inputting, on a membership registration screen provided by the server computer 11, information such as member organization name, user name, user password, e-mail address of the user, address for contact, and manner of payment for service fees.

In the present embodiment, the internal biological time information providing service is a service for members registered for membership in advance. However, services may be provided by selling a kit including a test tube for a blood sample 19 and an instruction manual for connecting to the server computer 11 in an information center 1 so as to allow a person who purchases the kit to have an one-time service.

The clients 2 to 7 draw blood from patients, place in test tubes provided by the information center 1, and send blood samples 19 to the information center 1. The information center 1 measures mRNA expression levels of the blood samples 19 with DNA chip readers 12 through a known method.

Upon completion of the expression level measurement of a blood sample 19, the information center 1 activates the server computer 11 to thereby start processing for circadian rhythm disorder determination, internal biological time estimation, and information provision.

The processing performed in the server computer 11 for determining on the presence or absence of circadian rhythm disorders, estimating internal biological time, and providing information will be described with reference to the flowcharts shown in FIGs. 10 to 13.

When the process is started, an initial setting is made in step S301. The initial setting process of step S301 will next be described in detail referring to the flowchart in FIG. 11.

In the initial setting process in step S301, firstly, in step S351, a threshold value D that has been input by a user of the server computer 11 through a keyboard 78 or the like is input and stored in an unillustrated threshold value memory area in the HDD 75.

The threshold value D is a decisive value for determining the presence of circadian rhythm disorders; if the maximum value C of the calculated Pearson's product moment of correlation coefficients between waves representing the expression levels of circadian oscillatory gene products in a sample and waves representing the estimated expression levels of the circadian oscillatory gene products is below the threshold value D, the presence of circadian rhythm disorders is determined.

Preferably, the threshold value D differs depending on the number of circadian oscillatory genes in the sample. For example, as threshold value D, a smaller value may be employed for a higher number of circadian oscillatory gene species contained in a sample. When the number of the circadian oscillatory gene species in the sample is about 100 to 200, a threshold value D of 0.3 or more, preferably 0.5 or more is employed.

In the present embodiment, the threshold value D is 0.5.

Subsequently, in step S352, a threshold value U entered through the keyboard 78 or the like by a user of the server computer 11 is input and stored in an unillustrated threshold value memory area in the HDD 75.

The threshold value U is a decisive value for determining the presence of circadian rhythm disorders; the value represents the time shift between the internal biological time as estimated for a circadian oscillatory gene in a sample and the environmental time. In the present embodiment, the threshold value U is 2 hours.

Next, in step S353, standard expression levels, standard variations, and the molecular times are input for each of the gene products of the standard specimens. In this step, molecular timetables corresponding to the type of the sample to be measured are selected from among the molecular timetables 15 generated through the circadian-expression-curve/molecular-timetable generation process (process 103 of FIG. 6) for each gene product, and stored in ROM 74.

Subsequently, in step S354, values of standard expression levels, standard variations, and molecular times are extracted from the molecular timetables stored in step S353 corresponding to the type of the sample to be measured, and the circadian expression curves of the standard specimens represented by the following equation are derived.

$$\alpha(t) = \frac{A}{B} Cos\left(\frac{2\pi(t - T)}{24}\right) + C \qquad (10)$$

where

A: standard variation of a circadian oscillatory gene A,
B: standard deviation of the cosine wave calculated from equations (4) and (5),
C: standard expression level of gene A,
T: molecular time of the cosine wave (for example, the following 144 different points in time in increments of 10 minutes; i.e., 0/60, 10/60, 20/60, 30/60, ..., 1420/60, and 1430/60), and
t: a certain point in time.

Subsequently, in step S355, the circadian expression curves are normalized. Specifically, a relative circadian expression curve represented by the following equation is derived by subtracting a standard expression level from a relevant circadian expression curve for each of the circadian oscillatory genes of the standard specimens, followed by division by standard variation.

$$\beta(t) = \frac{1}{B} Cos\left(\frac{2\pi(t - T)}{24}\right) \qquad (11)$$

where

> B: standard deviation of the cosine wave calculated from equations (4) and (5),
> T: molecular time of the cosine wave (for example, 144 different points in time in increments of 10 minutes; i.e., 0/60, 10/60, 20/60, 30/60, ..., 1420/60, and 1430/60), and
> t: a certain point in time.

The thus-obtained relative circadian expression curves are stored in ROM 74.

Through the above steps, the initial setting procedure in step S301 in FIG. 10 is terminated.

Subsequently, in step S302 in FIG. 10, the sampling time S of the sample of interest entered by a user on the server computer 11 is stored in RAM 73. The sampling time is an environmental time.

Next, in step S303, the circadian oscillatory gene product quantity data of the sample is registered in HDD 75. This step is performed by receiving the circadian oscillatory gene product quantity data of the sample from the DNA chip reader 12 operated by the user on the server computer 11.

Subsequently, in step S304, normalization of the circadian oscillatory gene product quantity data of the sample is performed, and relative expression levels for each of the circadian oscillatory gene products are determined. The process in step S304 will next be described in detail with reference to FIG. 12.

In step S361, the standard expression level of the standard specimens is subtracted from the circadian oscillatory gene product quantity data of the sample. The values of the standard expression level have been obtained through calculation of average values of circadian oscillatory gene product quantity data of the standard specimens for each of the genes.

In step S362, the values obtained in step S361 are divided by the relevant standard variation of the standard specimens. The values of the standard variation have been obtained through calculation of the standard deviation of circadian oscillatory gene product quantity data of the standard specimens for each of the genes.

Through the above steps, the normalization process in step S304 for the circadian oscillatory gene product quantity data is completed, and relative expression levels for each of the circadian oscillatory gene products of the sample are obtained.

Next, in step S305, a value 0 is substituted into t in equation $\beta(t)$ representing the relative circadian expression curves of the standard specimens, and values on the relative circadian expression curves for each of the circadian oscillatory genes at t=0 are computed.

Subsequently, in step S306, a determination is made as to whether or not the value substituted into t in equation $\beta(t)$ representing the relative circadian expression curves of the standard specimens is larger than 24.

If the value substituted into t in equation $\beta(t)$ representing the relative circadian expression curves of the standard specimens is not larger than 24 (step S306: NO), or in other words, if the value substituted into t in equation $\beta(t)$ representing the relative circadian expression curves of the standard specimens falls within a range of 0 to 24 inclusive, in step S307, Pearson's correlation coefficient c between a normalized circadian oscillatory gene product quantity and an estimated relative expression level of each of the circadian oscillatory gene products at time t is computed.

The process in step S307 will next be described in detail with reference to FIG. 13.

Firstly, in step S371, for each of the circadian oscillatory genes, an estimated relative expression level at time t on the relative circadian expression curve of the gene is obtained. In this process, the molecular timetable records for the respective circadian oscillatory genes are read one by one, and relative circadian expression curves for the circadian oscillatory genes are obtained. Subsequently, a value on a relative circadian expression curve is obtained at time t and employed as the estimated relative expression level. This process is performed for each and every circadian gene.

Subsequently, in step S372, the Pearson's product moment correlation coefficient between the relative expression levels of circadian oscillatory gene products of the sample at the sampling time which has been normalized in step S304 and the estimated relative expression levels at time t on the relative circadian expression curves for the circadian oscillatory genes is computed. Each computed value is stored in RAM 73 together with the value of time t, and the process shown in FIG. 13 is completed.

Subsequently, the process proceeds to step S308 in FIG. 10, and dt is added to the previous time t. The value t+dt is substituted into t in equation $\beta(t)$ which represents the relative circadian expression curves of the standard specimens, and the values on the relative circadian expression curves for each of the circadian oscillatory genes at t=t+dt are computed.

In the present embodiment, a value of 10 minutes or 10/60 hours is employed for dt.

Subsequently, the process proceeds to step S306, and a determination is made as to whether or not the value substituted into t in equation $\beta(t)$ representing the relative circadian expression curves of the standard specimens is larger than 24.

If the value substituted into t in equation $\beta(t)$ expressing the relative circadian expression curves of the standard specimens is larger than 24 (step S306: YES), calculation of Pearson's product moment correlation coefficient is considered to have been completed between the relative expression levels for the circadian oscillatory gene products of the sample and the estimated relative expression levels at every point in time between 0 and 24 on the relative circadian expression curves for the circadian oscillatory genes, and thus, the process proceeds to step S309, whereby the maximum value C of the Pearson's product moment correlation coefficients c and the internal biological time T which gives the maximum value C are retrieved.

In this step, a set of data for the Pearson's product moment correlation coefficients c stored in RAM 73 is read, and the maximum Pearson's product moment correlation coefficient value is extracted from the data set and employed as the maximum value C. Subsequently, the time corresponding to the maximum value C of the Pearson's product moment correlation coefficient is read and employed as the internal biological time T that gives the maximum value C. The maximum value C and the internal biological time T are registered in RAM 73 as a maximum value C and an internal biological time T of the sample.

Subsequently, in step S310, a determination is made as to whether the maximum value C stored in RAM 73 satisfies the following condition "C < threshold value D." Since a threshold value D of 0.5 has been registered in the present embodiment, in this step, a determination is made as to whether or not the maximum value C is smaller than 0.5.

If the maximum value C is smaller than the threshold value D (step S310: YES), then the relative expression levels of circadian oscillatory gene products of the sample at a certain time at which the sample was drawn do not exhibit similarity with any of the estimated relative expression levels calculated for the circadian oscillatory genes estimated at various times. Thus, the test subject from whom sample has been drawn is considered to have circadian oscillatory genes which do not exhibit normal circadian oscillations. In step S311, circadian rhythm disorder is diagnosed on this sample, and a value "1," which indicates the presence of rhythm disorders, is registered in circadian rhythm disorder presence/absence determination registry 167 of the corresponding entry number in the internal biological time information table 16.

If the condition "maximum value C < threshold value D" is not met (step S310: NO), or in other words, if the maximum value C is equal to or larger than the threshold value D, then there exists a time T at which the relative expression levels of circadian oscillatory gene products of the sample at a certain time at which the sample was drawn exhibits similarity with estimated relative expression levels estimated for the circadian oscillatory genes. Thus, the test subject from whom this sample has been drawn possibly has circadian oscillatory genes exhibiting normal circadian oscillations, and therefore, the process proceeds to step S312, and a determination is made as to whether the absolute value of the difference between internal biological time T and sampling time S satisfies the following relation.

$$|T - S| > \text{threshold value } U \tag{12}$$

Since a threshold value U of 2 hours has been registered, a determination is made as to whether the absolute value of the difference between the internal biological time T and the sampling time S is larger than 2 hours.

If the absolute value of the difference between the internal biological time T and the sampling time S is larger than the threshold value U (step S312: YES), the time shift from the sampling time S, which is an environmental time, is considered to be too large even though general errors which may be involved in the internal biological time estimation method of present invention are taken into account. Thus, in step S313, the presence of circadian rhythm disorders is diagnosed on this sample, and a value "1," which indicates rhythm disorders, is registered in circadian rhythm presence/absence determination registry 167 of the corresponding entry number in the internal biological time information table 16.

Subsequently, in step S314, the internal biological time T calculated in step S309 is stored in internal biological time estimation registry 168 under the corresponding entry number in the internal biological time information table 16, and the process is terminated.

If the absolute value of the difference between the internal biological time T and the sampling time S is not larger than the threshold value U (step S312: NO), or in other words, if the absolute value of the difference between the internal biological time T and the sampling time S is equal to or smaller than the threshold value U, the time shift from the sampling time S, which is an environmental time, is considered to fall within a normal range. In this case, value T is considered to be a reasonable internal biological time of the test subject, and the internal biological time T is registered in the internal biological time estimation registry 168 under the corresponding entry number in the internal biological time information table 16, and the process is terminated.

Through the above steps, the circadian rhythm disorder determination process and the internal biological estimation process are terminated.

A process for permitting clients 2 to 7 to have an access, by way of viewing, to information as to the presence or absence of circadian rhythm disorders or internal biological time information, which are stored in the server computer 11, will next be described.

Upon completion of the circadian-rhythm-disorder-determination/internal-biological-time-estimation stage 200 shown in FIG. 6, users of clients 2 to 7 who have sent the blood samples 19 of test subjects to the information center 1 receive from the server computer 11 e-mail notifying completion of the circadian rhythm disorder determination process and the internal biological estimation process.

After a user is advised of completion of the circadian rhythm disorder determination process and the internal biological estimation process through e-mail, the user receives a report 300, shown in FIG. 6, of internal biological time information on an internal biological time information report screen 91 provided by the server computer 11.

The process of FIG. 6 for reporting internal biological time information on the internal biological time information report screen 91 will next be described.

When the address of an internal biological time information providing service screen of the information center 1 is input via the Internet at the terminal computer 21, an unillustrated initial screen of the internal biological time information providing service is displayed.

In the initial screen, information on internal biological time and other general information items regarding drug dosage time management, etc. are displayed, as well as an ID/password input screen display button for the user to move to a "members only" screen for access to the internal biological time information providing service.

When the ID/password input screen display button is clicked, an ID/password input screen (not shown) is displayed. On this screen, when an ID and a password are input, the server computer 11 searches the internal biological information table 16 using the client ID number 162 for the client as a key item, extracts the records of the client, and generates data to be shown on the internal biological time information report screen 91.

Thereafter, the data to be shown on the internal biological time information report screen 91 is transmitted to the terminal computer 21.

When the user enters his or her membership ID on the "members only" screen provided by the terminal computer 21 for log-in, the internal biological time information report screen 91 as shown in FIG. 14 is displayed.

On the internal biological time information report screen 91, the following are displayed: an internal biological time information list 910 for the specimen on which the log-in member requested a determination on the presence or absence of rhythm disorders and an internal biological time estimation, a close button (or click box) 921 for closing the internal biological time information report screen 91, a download button 922 for downloading the determination or estimation results for a specimen check-marked in a download check box 919, and a log-out button 923.

The internal biological time information list 910 is a list prepared for each specimen and containing internal biological information of the specimen, and the following items are displayed for each specimen: entry number 911 of the specimen, test subject number 912 from whom the specimen has been drawn, sampling time 913 at which the specimen was drawn from the test subject, reception date 914 indicating the date on which the information center 1 received the specimen, the presence or absence of rhythm disorders 915 as a test result of the specimen, internal biological time information 916 as a test result of the specimen, a history screen display button 917 for the history regarding internal biological time information under the test subject number, a comment display button 918 for the test results of the specimen, and a download check box 919 for downloading the test results of the specimen.

By clicking the history screen display button 917, a user can view, in the list displayed, the history of the results as determined in the past on a test subject from whom the specimen has been drawn.

The server computer 11 may store a program for performing a data analysis on a large number of test subjects' internal biological time information data provided by the internal biological time estimation system.

This program may be configured so as to enable analysis regarding the correlation between internal biological time of a human subject and characteristics of that human subject through derivation of categorized internal biological time information by age, sex, ethnicity, pathological conditions, physical characteristics such as height and body weight, type of work, etc. The analysis results of correlation between human internal biological time and human characteristics may be utilized for human beings' health promotion and measures against diseases.

Next will be described the processing for data analysis on internal biological time information of a large number of test subjects obtained through provision of the internal biological time estimation system.

When the information center 1 receives a blood sample 19 along with a test subject number 165, the center 1 also receives characteristic data such as the date of birth, sex, ethnicity, pathological conditions, and height and body weight of the test subject.

These data may be received via the Internet 13 at the time of receipt of the blood sample 19. Alternatively, the blood sample 19 may be accompanied by a sheet of paper on which the data are shown.

The respective test subjects' characteristic data received by the information center 1 are stored in a test subject characteristic table 17 at the server computer 11, as shown in FIG. 15.

If the number of record items stored in the internal biological time information table 16 and the test subject characteristic table 17 has reached a predetermined number, the server computer 11 data starts data analysis.

Example data analysis results are shown in FIG. 16.

The results of data analysis may be used later for diagnosis of the sleep-wake rhythm disorders (delayed sleep phase syndrome, advanced sleep phase syndrome, non-24 hour circadian rhythm sleep-wake disorder, etc.), seasonal depression, and jet-lag syndrome.

No particular limitation is imposed on the type of the circadian expression curve, insofar as it can be expressed by a mathematical formula of a time-course expression profile of a circadian oscillatory gene derived on the basis of a finite number of expression data, or expression level data, which have been obtained over a certain period of time.

For example, the circadian expression curve may be generated through a method for creating a periodic curve, utilizing the Fourier transform, of time-course expression level data of each of the circadian oscillatory genes (Reference 1). The "Fourier transform" is a transform based on the principle first formulated by French mathematician-physicist Fourier, and is used for bridging two mathematical expressions (note: a physical process can be expressed either as a function of time; i.e., in the form of h(t), or as a function of frequency f; i.e., in the form of H(f)). The Fourier transform is represented by the following expressions:

$$h(t) = \int_{-\infty}^{\infty} H(f) \exp(-2\pi i f t) df \qquad (13)$$

$$H(f) = \int_{-\infty}^{\infty} h(t) \exp(2\pi i f t) dt \qquad (14)$$

The Fourier transform for a finite number of sample values (measurements) is called discrete Fourier transform. Suppose that there are a series of measurements in a number N which are to undergo a discrete Fourier transform:

$$h_k \equiv h(t_k), \ t_k \equiv k\Delta, \ k = 0,1,2,...,N-1$$

The sample interval is $\Delta$. Here, the discrete Fourier transform is defined as follows:

$$H_n \equiv \sum_{k=0}^{N-1} h_k \exp\left(\frac{2\pi i k n}{N}\right) \qquad (15)$$

where
n = 0,1,2,..., N - 1

By use of Hn, the following function of time, h(t), can be estimated:

$$h(t) \approx \frac{1}{N} \sum_{n=0}^{N-1} H_n \exp\left(-2\pi i \frac{n}{N\Delta} t\right) \qquad (16)$$

A circadian expression curve may alternatively be generated according to a method for creating an interpolation curve using an interpolation method, such as the spline interpolation (Reference 1). The "spline interpolation" is a method for interpolation used for generation of interpolation curves, finite element methods, function approximation, and extrapolation of experimental data, etc. (References 1 and 2).

Since a circadian expression curve obtained either by Fourier transform or by spline interpolation is expressed as a function of time, substitution of a certain time t into the function yields a value on the circadian expression curve, and if a corresponding standard expression level value is subtracted from the obtained value, followed by division by standard variation, a corresponding value on the relative expression curve can be calculated.

Estimation of internal biological time may be carried out as follows. In a preliminary step, relative circadian expression curves are generated for each of 6 or more, preferably 30 or more, more preferably 50 or more, particularly preferably 100 or more, arbitrary circadian oscillatory genes. The values, at time T, on these curves are compared with the relative expression levels of the corresponding circadian oscillatory gene products, which have been separately measured. The time showing the highest similarity in this comparison is estimated as the internal biological time. For example, in the case where circadian expression curves are generated in the form of cosine waves and similarity is calculated as the Pearson's product moment correlation coefficient, if the highest similarity shows correlation coefficient of 0.5 or higher, internal biological time can be estimated within an error range of 2 hours.

On the other hand, if sufficient similarity is not obtained between the two at all the times (e.g., Pearson's product moment correlation coefficient < 0.5), the presence of circadian rhythm disorders can be suggested. No particular limitation is imposed on the similarity, so long as it is a numeral value indicating similarity between an object and another object. Preferably, Pearson's product moment correlation coefficient is employed.

Circadian oscillatory genes used for estimating internal biological time may be known circadian oscillatory genes or genes extracted by use of the DNA chip method or the like (including genes whose functions are unknown), so long as they exhibit characteristics of the circadian oscillatory genes as described in the present specification.

Other than the methods of estimating internal biological time by defining similarity "a priori" as described above, internal biological time can be estimated by use of a learning algorithm, specifically, with a support vector machine, genetic algorithm, neural network, etc., for implementing similarity learning.

The following are references cited in the present specification:

Reference 1: "Numerical Recipes in C" William H. Press *et al.*, Gijutsuhyoron-sha; Reference 2: "Spline function using C parameters⎯Data Analysis, CG, and Differential Equation⎯" compiled under supervision by Akira SAKURAI, authored by Keisuke SUGANO, Kazumi YOSHIMURA, and Fumio TAKAYAMA, Tokyo Denki University Press; Reference 3: "Cluster Analysis and its Application" translated and supervised by Hideo NISHIDA, Uchida Rokakuho; and Reference 4: "Fundamental Statistics I - an Introduction to the Statistics" edited by The University of Tokyo, Komaba, College of Arts and Sciences (Statistics), published by University of Tokyo Press.

Industrial Availability

As described above, according to the present invention, there is provided internal biological time derivation means for obtaining internal biological time information including judgment as to whether or not a biological individual suffers a circadian rhythm disorder, and when the biological individual does not suffer a circadian rhythm disorder, an estimated internal biological time of the biological individual, through comparison between measurement data and above-described circadian expression curves specified by the above-described molecular timetables. Therefore, a comparison of assay data on a specimen sampled at a single point in time with the above-mentioned circadian expression curves specified by molecular timetables generated for a predetermined portion of a predetermined biological species achieves, without need for sampling specimens a plurality of times, a simple judgment procedure for determining, on the basis of a specimen sampled at a single point in time, as to the presence or absence of a circadian rhythm disorder, and for estimating the internal biological time.

**Claims**

1. A molecular timetable generating apparatus for estimating internal biological time of a biological individual on the basis of gene product quantity data as measured in standard specimens sampled from individuals, the apparatus being **characterized by** comprising:

    data input means for inputting gene product quantity data of standard specimens each sampled from a predetermined portion of each of a plurality of individuals of a predetermined biological species;
    circadian oscillatory gene selection means for selecting, from among the genes which are expressed in the aforementioned standard specimens, circadian oscillatory genes whose time-course change in gene product quantity data approximates a cosine curve having a predetermined period;
    circadian expression curve selection means for selecting, from among a plurality of cosine curves having different phases and sharing a specific period, a circadian expression curve which is similar to the pattern of time-course change in the expression product quantity of each of the above-selected circadian oscillatory genes; and

registration means for registering information which identifies the selected circadian expression curve.

2. The molecular timetable generating apparatus as recited in claim 1, **characterized in that** the registration means is configured so as to register, as a standard molecular time of the circadian oscillatory gene, a point in time at which the circadian expression curve reaches the maximum, in the molecular timetable used for estimating the internal biological time, and also to register, in the molecular timetable, an average value and standard deviation, both calculated for each circadian oscillatory gene, of the expression product quantity data, as a standard expression quantity and standard variation of the circadian oscillatory gene, respectively.

3. An internal biological time estimation apparatus for estimating internal biological time of a biological individual on the basis of gene product quantity data as measured in specimens sampled from individuals, the apparatus comprising:

molecular timetable storage means for storing molecular timetables each of which specifies a circadian expression curve showing time-course change in the expression product quantity of a circadian oscillatory gene contained in a predetermined portion of a predetermined biological species;
data input means for inputting the gene product quantity data of the circadian oscillatory genes contained in a specimen sampled from said predetermined portion of the biological individual; and
internal biological time derivation means for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable.

4. A molecular timetable generating method which enables, by use of information processing equipment, generation of a molecular timetable for estimating internal biological time of a biological individual on the basis of gene product quantity data as measured in standard specimens sampled from individuals, the method comprising:

a data input procedure for inputting gene product quantity data of standard specimens each sampled from a predetermined portion of each of a plurality of individuals of a predetermined biological species;
a circadian oscillatory gene selection procedure for selecting, from among the genes which are expressed in the aforementioned standard specimens, circadian oscillatory genes whose time-course change in gene product quantity data approximates a cosine curve having a predetermined period;
a circadian expression curve selection procedure for selecting, from among a plurality of cosine curves having different phases and sharing a specific period, a circadian expression curve which is similar to the pattern of time-course change in the expression product quantity of each of the above-selected circadian oscillatory genes; and
a registration procedure for registering information which identifies the selected circadian expression curve.

5. An internal biological time estimation method for estimating, by use of information processing equipment, internal biological time of a biological individual on the basis of gene product quantity data as measured in specimens sampled from individuals, the method being **characterized by** comprising:

a molecular timetable storage procedure for storing molecular timetables each of which specifies a circadian expression curve showing time-course change in the expression product quantity of a circadian oscillatory gene contained in a predetermined portion of a predetermined biological species;
a data input procedure for inputting the gene product quantity data of the circadian oscillatory genes contained in a specimen sampled from said predetermined portion of the biological individual; and
an internal biological time derivation procedure for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable.

6. A molecular timetable generating program which enables information processing equipment to perform:

a data input procedure for inputting gene product quantity data of standard specimens each sampled from a predetermined portion of each of a plurality of individuals of a predetermined biological species;

a circadian oscillatory gene selection procedure for selecting, from among the genes which are expressed in the aforementioned standard specimens, circadian oscillatory genes whose time-course change in gene product quantity data approximates a cosine curve having a predetermined period;

a circadian expression curve selection procedure for selecting, from among a plurality of cosine curves having different phases and sharing a specific period, a circadian expression curve which is similar to the pattern of time-course change in the expression product quantity of each of the above-selected circadian oscillatory genes; and

a registration procedure for registering information which identifies the selected circadian expression curve; said information processing equipment being provided for generation of a molecular timetable for estimating internal biological time of the biological individual on the basis of gene product quantity data as measured in standard specimens sampled from individuals.

**7.** An internal biological time estimation program which enables information processing equipment to perform:

a molecular timetable storage procedure for storing molecular timetables each of which specifies a circadian expression curve showing time-course change in the expression product quantity of a circadian oscillatory gene contained in a predetermined portion of a predetermined biological species;

a data input procedure for inputting the gene product quantity data of the circadian oscillatory genes contained in a specimen sampled from said predetermined portion of the biological individual; and

an internal biological time derivation procedure for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable;

said information processing equipment being provided for estimating internal biological time of the biological individual on the basis of gene product quantity data as measured in standard specimens sampled from individuals.

**8.** An internal biological time estimation system which enables generation of a molecular timetable for estimating internal biological time of a biological individual on the basis of gene product quantity data as measured in standard specimens sampled from individuals and estimation of internal biological time of the biological individual on the basis of gene product quantity data as measured in a specimen sampled from the biological individual; said system being **characterized by** comprising:

a server computer installed in an information center for providing internal biological time information, and a terminal computer connected to the server computer so as to transmit and receive information therebetween,

wherein the server computer includes:

standard data input means for inputting gene product quantity data of standard specimens each sampled from a predetermined portion of each of a plurality of individuals of a predetermined biological species;

circadian oscillatory gene selection means for selecting, from among the genes which are expressed in the aforementioned standard specimens, circadian oscillatory genes whose time-course change in gene product quantity data approximates a cosine curve having a predetermined period;

circadian expression curve selection means for selecting, from among a plurality of cosine curves having different phases and sharing a specific period, a circadian expression curve which is similar to the pattern of time-course change in the expression product quantity of each of the above-selected circadian oscillatory genes;

registration means for registering, as a standard molecular time of the circadian oscillatory gene, a point in time at which the circadian expression curve reaches the maximum, in the molecular timetable used for estimating the internal biological time, and also for registering, in the molecular timetable, an average value and standard deviation, both calculated for each circadian oscillatory gene, of the expression product quantity data, as a standard expression quantity and standard variation of the circadian oscillatory gene, respectively;

measurement data input means for inputting the gene product quantity data of the circadian oscillatory genes contained in the specimen sampled from the said predetermined portion of the biological individual;

internal biological time derivation means for obtaining internal biological time information including judgment as to whether or not the biological individual suffers a circadian rhythm disorder, and when the biological individual does not suffer a circadian rhythm disorder, an estimated internal biological time of the biological individual, through comparison between the input measurement data and the circadian expression curve specified by the molecular timetable; and

internal biological time transmission means for transmitting the obtained internal biological time information to the terminal computer.

# Fig. 1

INFORMATION CENTER — 11

13

1

EDUCATIONAL
INSTITUTION — 7

12

19

MEDICAL FACILITY

71 — 72

CORPORATE MEMBER — 6

2 — 22 — 21

61 — 62

3 — 32 — 31

SPORTS CENTER

4

INDIVIDUAL
MEMBER

41

51 — 52

5

HEALTH CARE
FACILITY FOR
THE ELDERLY

Fig. 2

| PRINTER (81) | MOUSE (79) | KEYBOARD (78) | DISPLAY (80) | MEDIUM STORAGE DEVICE (76) | COMMUNI-CATION DEVICE (77) | (13) |

CPU (70)

| RAM (73) | ROM (74) | HDD (75) |

# Fig. 3

TIME-COURSE GENE PRODUCT QUANTITY TABLE

| NUMBER | ACCESSION NUMBER | ZT=0 | ZT=4 | ZT=8 | ZT=12 | ZT=16 | ZT=20 | ZT=24 | ZT=28 | ZT=32 | ZT=36 | ZT=40 | ZT=44 |
|--------|------------------|------|------|------|-------|-------|-------|-------|-------|-------|-------|-------|-------|
| 1 | AA_00000 | | | | | | | | | | | | |

MOLECULAR TIMETABLE

| NUMBER | ACCESSION NUMBER | STANDARD EXPRESSION LEVEL (LD) | STANDARD VARIATION (LD) | MOLECULAR TIME (LD) | STANDARD EXPRESSION LEVEL (DD) | STANDARD VARIATION (DD) | MOLECULAR TIME (DD) |
|---|---|---|---|---|---|---|---|
| 1 | AA_00000 | 1126 | 902 | 9:10 | 984 | 572 | 8:30 |

# Fig. 5

INTERNAL BIOLOGICAL TIME INFORMATION TABLE

| ENTRY NUMBER | CLIENT ID NUMBER | USER ID | USER PASSWORD | TEST SUBJECT NUMBER | COLLECTION TIME | RHYTHM DISORDERS | INTERNAL BIOLOGICAL TIME | COMMENT |
|---|---|---|---|---|---|---|---|---|
| 028010008 | A12345 | A12345BB | USERAA | A000345 | 2002/08/01/ 08 | 0 | 08:52 | |

## Fig. 6

```
                                                            ┌─────────────────────┐ 101
                                                            │ TIME-COURSE GENE    │
                                                            │ PRODUCT             │
                                                            │ QUANTITY DATA OF    │
                                                            │ STANDARD            │
                                                            │ SPECIMENS           │
                                                            └─────────────────────┘
                                                                     │
                ┌─────────────────────┐                              ▼        102
                │ TIME-COURSE GENE    │                    ┌─────────────────────┐
                │ PRODUCT QUANTITY    │                    │ CIRCADIAN           │
                │ DATA OF SAMPLE      │                    │ OSCILLATION         │
                └─────────────────────┘                    │ GENE SELECTION      │
                          │                                └─────────────────────┘
                          ▼                                         │
  ┌──────────┐   YES    ◇─────────────◇                             ▼
  │ RHYTHM   │◄────────│ RHYTHM       │          ┌─────────────────────────────┐
  │ DISORDERS│         │ DISORDER     │◄─────────│ CIRCADIAN EXPRESSION CURVE  │
  └──────────┘         │ DETECTION    │          │ AND MOLECULAR TIMETABLE     │
        │              ◇─────────────◇           │ GENERATION FOR              │
        │                 │ NO                    │ EACH GENE PRODUCT           │
        │                 ▼                       └─────────────────────────────┘ 103
        │         ┌─────────────────┐                       │
        │         │ INTERNAL        │◄──────────────────────┘
        │         │ BIOLOGICAL      │
        │         │ TIME ESTIMATION │
        │         └─────────────────┘
        │                 │
        ▼                 ▼
  ┌──────────┐    ┌──────────┐
  │ REPORT   │◄───│ INTERNAL │
  └──────────┘    │ BIOLOGICAL│
                  │ TIME      │
                  └──────────┘
```

300

CIRCADIAN RHYTHM DISORDER
DETERMINATION/INTERNAL
BIOLOGICAL TIME ESTIMATION

200

GENE SELECTION AND
TIMETABLE GENERATION

100

Fig. 7

START

READ ONE
GENE RECORD — S101

SUBSTITUTE
PERIOD AND T
INTO Z(t) — S102

CALCULATE AND
STORE PRODUCT
MOMENT
CORRELATION
COEFFICIENT — S103

PERIOD OR T
REMAINING? — S104

Yes

No

0.75 <
COEFFICIENT? — S105

No

Yes

SET GENE
CANDIDATE FLAG — S106

GENE RECORD
REMAINING? — S107

Yes

No

GENERATE
CIRCADIAN
OSCILLATION
GENE TABLE — S108

END

Fig. 8

Fig. 9

START

READ ONE CIRCADIAN GENE AND CONDITION RECORD — S201

SUBSTITUTE T INTO $\alpha(t)$ — S202

CALCULATE AND STORE PRODUCT MOMENT CORRELATION COEFFICIENT — S203

T REMAINING? — S204
Yes

No

REGISTER COSINE WAVE RECORD FOR LARGEST COEFFICIENT — S205

REGISTER MOLECULAR TIME, STANDARD EXPRESSION LEVEL, AND STANDARD VARIATION IN MOLECULAR TIMETABLE — S206

CIRCADIAN GENE OR CONDITION REMAINING? — S207
Yes

No

END

# Fig. 10

START

INITIAL SETTING — S301

REGISTER SAMPLING TIME S (ENVIRONMENTAL TIME) — S302

REGISTER CIRCADIAN GENE PRODUCT QUANTITY OF SAMPLE — S303

NORMALIZE CIRCADIAN GENE PRODUCT QUANTITY OF SAMPLE — S304

SUBSTITUTE 0 FOR t INTO $\beta(t)$ — S305

S306 — t > 24

No

S307 — CALCULATE PEARSON'S CORRELATION COEFFICIENT c BETWEEN NORMALIZED CIRCADIAN OSCILLATION GENE PRODUCT QUANTITY AND ESTIMATED PRODUCT QUANTITY FOR EACH CIRCADIAN OSCILLATION GENE AT TIME t

Yes

S309 — SEARCH MAXIMUM VALUE C OF PEARSON'S CORRELATION CO-EFFICIENT c AND INTERNAL BIOLOGICAL TIME T CORRES-PONDING TO MAXIMUM VALUE C

SUBSTITUTE t+dt FOR t INTO $\beta(t)$ — S308

S310 — C < THRESHOLD VALUE D?

Yes

S311 — REGISTER AS RHYTHM DISORDER

No

S312 — |T-S| > THRESHOLD VALUE U?

Yes

REGISTER AS RHYTHM DISORDER — S313

No

S314 — REGISTER INTERNAL BIOLOGICAL TIME T

END

# Fig. 11

```
        ┌─────────────────────────┐
        │     INITIAL SETTING     │
        └─────────────────────────┘
                     │                         S351
                     ▼
        ┌─────────────────────────┐
        │  INPUT THRESHOLD VALUE D │
        └─────────────────────────┘
                     │                         S352
                     ▼
        ┌─────────────────────────┐
        │  INPUT THRESHOLD VALUE U │
        └─────────────────────────┘
                     │                         S353
                     ▼
        ┌─────────────────────────┐
        │ INPUT STANDARD EXPRESSION│
        │ LEVEL, STANDARD VARIATION,│
        │ AND MOLECULAR TIME FOR   │
        │ EACH GENE PRODUCT        │
        └─────────────────────────┘
                     │                         S354
                     ▼
        ┌─────────────────────────┐
        │ DERIVE CIRCADIAN EXPRESSION│
        │ CURVE (A SET OF ESTIMATED │
        │ EXPRESSION LEVELS FOR EACH │
        │ GENE PRODUCT AT INTERNAL  │
        │ BIOLOGICAL TIME t)        │
        └─────────────────────────┘
                     │                         S355
                     ▼
        ┌─────────────────────────┐
        │    NORMALIZE/REGISTER    │
        │ CIRCADIAN EXPRESSION CURVE│
        └─────────────────────────┘
                     │
                     ▼
        ┌─────────────────────────┐
        │           END           │
        └─────────────────────────┘
```

## Fig. 12

Flowchart:

NORMALIZATION OF CIRCADIAN OSCILLATION GENE PRODUCT QUANTITY OF SAMPLE

↓

SUBTRACT STANDARD EXPRESSION LEVEL FROM CIRCADIAN OSCILLATION GENE PRODUCT QUANTITY OF SAMPLE — S361

↓

DIVIDE BY STANDARD VARIATION — S362

↓

END

# Fig. 13

```
┌─────────────────────────────┐
│  CALCULATE PEARSON'S        │
│  CORRELATION COEFFICIENT c  │
│  BETWEEN NORMALIZED CIRCADIAN│
│  OSCILLATION GENE PRODUCT   │
│  QUANTITY AND ESTIMATED     │
│  RELATIVE EXPRESSION LEVELS FOR│
│  EACH CIRCADIAN OSCILLATION │
│  GENE PRODUCT AT TIME t     │
└─────────────────────────────┘
              │
              ▼
┌─────────────────────────────┐   ⟋S371
│  OBTAIN ESTIMATED RELATIVE  │
│  EXPRESSION LEVELS          │
│  ON RELATIVE CIRCADIAN      │
│  EXPRESSION CURVE FOR EACH  │
│  CIRCADIAN OSCILLATION GENE │
│  AT TIME t                  │
└─────────────────────────────┘
              │
              ▼
┌─────────────────────────────┐   ⟋S372
│  CALCULATE PEARSON'S        │
│  CORRELATION COEFFICIENT c  │
│  BETWEEN NORMALIZED         │
│  CIRCADIAN OSCILLATION GENE │
│  PRODUCT QUANTITY AND ESTIMATED│
│  RELATIVE EXPRESSION LEVELS ON │
│  RELATIVE CIRCADIAN EXPRESSION │
│  CURVE FOR EACH             │
│  CIRCADIAN OSCILLATION      │
│  GENE PRODUCT AT TIME t     │
└─────────────────────────────┘
              │
              ▼
         (   END   )
```

## Fig. 14

911  912  913  914  916  917  91

CLIENT NAME: HOSPITAL A

USER NAME: A. A.

INTERNAL BIOLOGICAL TIME INFORMATION REPORT

| | ENTRY NUMBER | TEST SUBJECT | SAMPLING TIME | DATE RECEIVED | RHYTHM DIS- ORDERS | INTERNAL BIOLOGI- CAL TIME | HISTORY | COMMENT | DOWNLOAD | ▲ |
|---|---|---|---|---|---|---|---|---|---|---|
| 1 | 0208010008 | A000345 | 02/08/01 08:00 | 02/08/02 | 0 | 8:52 | HISTORY | COMMENT | ☐ | |
| 2 | | | | | | | | | | |
| 3 | | | | | | | | | | |
| 4 | | | | | | | | | | |
| 5 | | | | | | | | | | |
| 6 | | | | | | | | | | |
| 7 | | | | | | | | | | |

CLOSE    DOWNLOAD    LOGOUT

915   918  919  910

921   922   923

# Fig. 15

INTERNAL BIOLOGICAL TIME INFORMATION TABLE

| CLIENT ID NUMBER | TEST SUBJECT NUMBER | USER ID | USER PASSWORD | DATE OF BIRTH | SEX | ETHNICITY | HISTORY OF DISEASE | HEIGHT | BODY WEIGHT |
|---|---|---|---|---|---|---|---|---|---|
| A12345 | A000345 | A12345B B | USERAA | 1950/05/0 1 | MALE | JAPANESE | NONE | 170 cm | 60 kg |

## Fig. 16

|  | 10 – 30 YEARS OLD | 30 – 50 YEARS OLD | 50 – 70 YEARS OLD |
|---|---|---|---|
| CIRCADIAN RHYTHM DISORDERS | 1 PERSON | 7 PERSONS | 15 PERSONS |
| AVERAGE INTERNAL BIOLOGICAL TIME | -20 MINUTES | +20 MINUTES | +1 HOUR 20 MINUTES |
| TOTAL NUMBER OF TEST SUBJECTS | 50 PERSONS | 50 PERSONS | 50 PERSONS |

## INTERNATIONAL SEARCH REPORT

| International application No. |
| --- |
| PCT/JP03/09579 |

**A. CLASSIFICATION OF SUBJECT MATTER**
Int.Cl$^7$ G06F19/00

According to International Patent Classification (IPC) or to both national classification and IPC

**B. FIELDS SEARCHED**

Minimum documentation searched (classification system followed by classification symbols)
. Int.Cl$^7$ G06F19/00

Documentation searched other than minimum documentation to the extent that such documents are included in the fields searched

| | | | |
| --- | --- | --- | --- |
| Jitsuyo Shinan Koho | 1922–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)
JICST FILE(JOIS)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | Adam CLARIDGE-CHANG, Circadian Regulation of Gene Expression Systems in the Drosophila Head, Neuron, 20 November, 2001 (20.11.01), Vol.32, No.4, pages 657 to 671 | 1-2,4,6<br>3,5,7-8 |
| A | JP 4-506020 A (Brigham and Women's Hospital), 22 October, 1992 (22.10.92), Full text; Figs. 1 to 41<br>& WO 90/15639 A1 & US 5176133 A | 3,5,7-8 |

☐ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>14 October, 2003 (14.10.03) | Date of mailing of the international search report<br>28 October, 2003 (28.10.03) |
| --- | --- |
| Name and mailing address of the ISA/<br>Japanese Patent Office | Authorized officer |
| Facsimile No. | Telephone No. |

Form PCT/ISA/210 (second sheet) (July 1998)